# EUROPÄISCHE PATENTSCHRIFT

(11) **EP 0 648 138 B1**
(45) Veröffentlichungstag und Bekanntmachung des Hinweises auf die Patenterteilung: **11.12.1996**
(21) Anmeldenummer: 93915765.7
(22) Anmeldetag: 01.07.1993
(51) Int. Cl.: A61M 16/04

(54) **TRACHEAL- ODER TRACHEOSTOMIE-TUBUS UND DAMIT AUSGESTATTETE BEATMUNGSANLAGEN**
TRACHEAL OR TRACHEOTOMY TUBE AND BREATHING INSTALLATIONS FITTED THEREWITH
TUBE TRACHEAL OU DE TRACHEOTOMIE ET DISPOSITIF RESPIRATOIRE ASSOCIE

(30) Priorität: 03.07.1992 DE 4221931
(43) Veröffentlichungstag der Anmeldung: 19.04.1995
(73) Patentinhaber: MANG, Harald, 91096 Möhrendorf (DE)
(72) Erfinder: MANG, Harald, 91096 Möhrendorf (DE)
(74) Vertreter: Müller-Gerbes, Margot, Dipl.-Ing.
(86) Internationale Anmeldenummer: EP9301700
(87) Internationale Veröffentlichungsnummer: WO9401157

(56) Entgegenhaltungen:
- EP-A- 0 175 096
- EP-A- 0 366 524
- AT-B- 389 818
- DE-A- 2 535 191
- US-A- 4 715 848

## Beschreibung

Die Erfindung bezieht sich auf einen Tubus zum Einführen in die Atemwege eines Patienten insbesondere orotracheale oder nasotracheale Tuben oder Tracheostomie-Tuben für eine steuerbare Beatmung eines Patienten mit einem distalen in die Atemwege einführbaren Ende und einem proximalen mit Anschlüssen versehenen Ende und zwei im wesentlichen vom proximalen bis zum distalen Ende des Tubus durchgehenden Lumina, von denen das eine der beiden Lumen als Respirationslumen bei der Beatmung oder Atmung und das andere der beiden Lumen als Inspirationslumen der zusätzlichen oder alleinigen Zufuhr von Atemgas dient.

Tracheal- und Tracheostomie-Tuben werden für die künstliche Beatmung eines Menschen in Verbindung mit Beatmungsgeräten, umfassend zumindest ein Exspirationsventil und eine Atemgasquelle, eingesetzt. Die Erfindung befaßt sich daher in Weiterbildung des Tubus mit einer Beatmungsanlage zur Verbesserung der Beatmung und zur Verringerung des in den Atemwegen verbleibenden Restatemgases unter Einsatz eines in seinen Funktionen verbesserten Tubus.

Die künstliche Beatmung eines Menschen erfolgt in der Regel in Übereinstimmung mit der natürlichen Atemfrequenz. Bei den verschiedenen verfahren der Hochfrequenz-Ventilation werden auch Beatmungsfrequenzen im Bereich der Herzfrequenz benutzt, ohne daß eine Synchronisierung der Beatmungsphasen mit der Herzaktion vorgenommen wird.

Die bisher bekannten konventionellen Beatmungsgeräte mit steuerbarem Exspirationsventil und steuerbarer Atemgaszufuhr mittels eines elektrisch steuerbaren Druckminderventiles erfordern einen einlumigen Tubus (Beatmungsrohr) oder einen doppellumigen Tubus (Inspirationslumen, Exspirationslumen), die so gestaltet sind, daß der Atemgasfluß in einem Lumen nicht auf den des anderen Lumens einwirkt, siehe DE 25 35 191 A1, AT 389 818. Die bekannten Beatmungsgeräte sind praktisch nur über eine Stauung der Atemgasquelle beeinflußbar, wobei bei jedem Zyklus ein Rest des Atemgases im Tubus verbleibt, was sich nachteilig auswirkt.

Aufgabe der Erfindung ist es, diesen Nachteil von Beatmungsanlagen mit doppellumigen Tuben zu beseitigen. Darüber hinaus soll die Steuerung der Beatmung in Abhängigkeit von Signalen, wie sie durch einen Elektrokadiographen oder z.B. einen Blutdruckmonitor vorliegen, ermöglicht werden.

Zur Lösung dieser Aufgabe wird erfindungsgemäß die Ausgestaltung des Tubus gemäß Gattungsbegriff des Patentanspruches 1 in der Weise vorgeschlagen, daß jedes der beiden Lumen eine distale Austrittsöffnung aufweist und die distale Austrittsöffnung des Inspirationslumens auf die distale Austrittsöffnung des Respirationslumens gerichtet ist. Bei einer bevorzugten Ausbildung des Tubus ist das Inspirationslumen im Bereich des distalen Endes des Tubus um etwa 180° umgelenkt und auf die bzw. in die distale Austrittsöffnung des Respirationslumens und durch das Respirationslumen zum proximalen Ende des Tubus gerichtet. Vorzugsweise weist bei dem erfindungsgemäßen Tubus eines der beiden Lumen einen kleineren Querschnitt als das andere der beiden Lumen des Tubus auf und das Lumen mit gegenüber dem anderen Lumen größeren Querschnitt als Respirationslumen für die Beatmung und Atmung dient und das Lumen mit dem gegenüber dem anderen Lumen kleineren Querschnitt als Inspirationslumen für die zusätzliche oder alleinige Atemgaszufuhr dient.

Der erfindungsgemäße Tubus, der als orotrachealer, nasotrachealer oder Tracheostomie-Tubus ausgebildet sein kann, weist zwei Lumina auf, die so beschaffen sind, daß über das Respirationslumen eine konventionelle Beatmung durchgeführt werden kann (z.B. intermittierende Überdruckbeatmung, intermittent positive pressure ventilation, IPPV). Ferner kann über dieses Lumen endotracheal und endobronchial abgesaugt werden sowohl mit handelsüblichen Einmal-Absaugkathetern als auch mit einem Bronchoskop. Das gegenüber dem Respirationslumen mit kleinerem Querschnitt ausgebildete Inspirationslumen ist für eine zusätzliche oder alleinige Atemgaszufuhr zum Patienten gedacht. Sowohl die zusätzliche als auch die alleinige Gaszufuhr über das Inspirationslumen können zeitweise, vorzugsweise exspiratorisch oder ständig oder kontinuierlich, aber in Abhängigkeit von der Atemphase mit unterschiedlicher Stromstärke, auch als Fluß oder Flow bezeichnet, erfolgen. Das distale Ende des Tubus, das in der Luftröhre zu liegen kommt, ist so gestaltet, daß sich das Inspirationslumen düsenförmig verengt und seine Öffnung oder Öffnungen auf bzw. in das offene distale Austrittsende des Respirationslumens weisen bzw. hineinreichen. Bildlich gesprochen, "bläst" das düsenförmig ausgebildete distale Ende des Inspirationslumens in die Austrittsöffnung des Respirationslumens und zwar in Richtung der Ausatmung durch das Respirationslumen auf das proximale Ende des Tubus hin, d.h. aus der Luftröhre und damit aus dem Patienten nach draußen. Am proximalen Ende des Tubus außerhalb der Luftröhre besitzen beide Lumina des Tubus Anschlüsse (Konnektoren) für die Verbindungsleitungen zu den jeweils verwendeten Atemgasquellen, Ventilen oder sonstigen Geräten.

Durch die erfindungsgemäße Ausbildung des distalen Endes des Inspirationslumens, das der Atemgaszufuhr dient, in Gestalt einer Düse, wird das über das Inspirationslumen heranströmende Atemgas um 180° umgelenkt, so daß es von dem distalen Ende des Tubus her in das Respirationslumen des Tubus in Richtung auf dessen proximales Endes zu hineingeleitet wird und zum proximalen Ende hinströmt. Zur Erzielung eines Jet-Effektes nach dem Strahlpumpenprinzip verringert sich die Querschnittsfläche des Inspirationslumens des Tubus am distalen Ende bis zur Düsenaustrittsöffnung vorzugsweise auf etwa die Hälfte.

Wenn bei der Beatmung eines Patienten der erfindungsgemäße Tubus eingesetzt wird, kann durch kontinuierliche Zufuhr von Atemgas durch das kleinere Lumen der Totraum bei der Beatmung erheblich verringert werden, d.h. die Menge des nicht ausgeatmeten Restgases wird erheblich verringert. Mit dem erfindungsgemäßen Tubus ist es somit möglich, zur Verbesserung der Ventilation bei der Beatmung eines Patienten die Kohlendioxidelimination durch Reduktion des Totraumes zu steigern, indem über das kleinere Lumen des Tubus zusätzlich ein kontinuierlicher retrograder oder exspiratorisch retrograder Atemgasfluß gegeben wird.

Bei den erfindungsgemäßen Tuben mit zwei Lumen unterschiedlicher Größe soll das größere Lumen sowohl eine ungehinderte konventionelle Beatmung als auch eine Spontanatmung des Patienten erlauben. Des weiteren soll das größere Lumen die Bronchialtoilette mit Absaugkathetern oder mit einem Bronchoskop ermöglichen. Das kleinere Lumen des Tubus dient der Atemgaszufuhr. Seine Querschnittsfläche ist dabei so zu wählen, daß in jeder patientgerechten Tubusgröße die Zufuhr bis zum 5-fachen des spontanen Atemminutenvolumens möglich ist, wobei Drücke, wie sie hinter handelsüblichen den Atemgasquellen zugeordneten Durchflußreglern herrschen, vorausgesetzt sind.

Als Richtlinie seien beispielhaft die folgenden Querschnittsverhältnisse angegeben: Größeres Lumen (Respirationslumen) zu kleinerem Lumen (Inpsirationslumen) zu Düsenaustrittsöffnung wie 8:2:1. Bei Tuben für Erwachsene soll die Querschnittsfläche des größeren Lumens wenigstens der eines konventionellen Tubus mit 7 mm Innendurchmesser entsprechen, d.h. ca. 38,5 mm² betragen. Das kleinere Lumen des Tubus entspricht dann mit 9,6 mm² einem Innendurchmesser eines konventionellen Tubus mit 3,5 mm Innendurchmesser. Die Querschnittsfläche der Düsenaustrittsöffnung des kleineren Lumens beträgt in diesem Beispiel dann 4,8 mm².

Vorteilhafte Ausgestaltungen des erfindungsgemäßen Tubus sind den kennzeichnenden Merkmalen der Unteransprüche 2 bis 21 entnehmbar.

Die Tuben können ohne oder mit Manschette gebaut werden. Die Manschette kann aufblasbar (Ballon) sein oder sich selbst entfalten (sog. "foam cuff"). Wenn keine Manschette vorgesehen ist, können sogenannte "gills", das sind sich nach Einführung des Tubus selbst aufrichtende, hauchdünne kreisrund um den Tubusschaft angeordnete Membranen oder Kiemen aus Silikon zur Abdichtung Verwendung finden.

Die Tuben selbst werden aus einem körperverträglichen Kunststoff, z.B. PVC, vorzugsweise aus Kunststoffen, die sich im Körper auch bei längerer Liegedauer nicht verändern und nach Gebrauch umweltfreundlich entsorgen lassen, hergestellt. Es können auch spiralarmierte Tuben aus Kautschuk oder Silikon (nach Woodbridge) verwendet werden. Zur optimalen Ausnutzung der Querschnittsfläche des Tubus soll seine Wandstärke möglichst gering sein.

Zur Erzielung eines Jet-Effektes mit Hilfe der am distalen Ende des Inspirationslumens ausgebildeten Düse sowie der Umlenkung der Strömungsrichtung in dem Inspirationslumen in Richtung des Respirationslumens, eintretend am distalen Ende desselben, kann die Düsenaustrittsöffnung des Inspirationslumens etwas in die Austrittsöffnung, d.h. in das Respirationslumen hineinragen, oder vor der Austrittsöffnung des Respirationslumens angeordnet sein oder direkt aneinandergrenzend. Die Zuordnung der Austrittsöffnung des Respirationslumens und der Düsenaustrittsöffnung des Inspirationslumens soll ausreichend freien Raum zum Mitreißen des Ausatmungsgases lassen, gleichzeitig jedoch auch die Austrittsöffnung des Respirationslumens nicht versperren, so daß sowohl eine ungehinderte Strömung bei der Beatmung möglich ist als auch beispielsweise ein Absaugkatheter durch das Respirationslumen des Tubus hindurchführbar ist.

Zur Erzielung eines Venturi-Effektes, der die aus den Atemwegen mitgerissene Gasmenge erhöht, kann sich im patientennahen (distalen) Eingangsbereich des Respirationslumens ein Wulst (kurzstreckige Verringerung der Querschnittsfläche bis zu etwa 25 %) befinden.

Der die Düse am patientennahen distalen Tubusende des Inspirationslumens bildende Düsenformkörper besteht ebenfalls aus einem gewebeverträglichen und in der Entsorgung umweltfreundlichen Kunststoff, wobei vorzugsweise ein härteres Material als für den Tubus selbst zur Verwendung kommt. Die Düse bzw. der Düsenformkörper erhält einen definierten Sitz im Verhältnis zum Tubus, z.B. durch einen Schnappverschluß; sie wird außerdem mit dem Tubus verklebt oder verschweißt.

Der erfindungsgemäße doppellumige Tubus mit einer Düse am distalen Ausgang des Inspirationslumens, die in das Respirationslumen vom distalen Ende desselben her hineinbläst, kann zur Unterstützung der Beatmung mit einem kontinuierlichen retrograden Atemgasfluß oder einem exspiratorischen retrograden Atemgasfluß oder zur Beatmung mit einem kontinuierlichen retrograden Atemgasfluß eingesetzt werden. Atemgas kann hierbei Luft oder Sauerstoff, geliefert aus einer Atemgasquelle, die zusätzlich mit einem Durchflußmesser für die Druckluft oder den Sauerstoff ausgerüstet ist, geliefert werden. Die maschinelle Beatmung benötigt neben dem doppellumigen Tubus, der in die Luftröhre eines Patienten eingesetzt wird, und der Atemgasquelle noch ein steuerbares Exspirationsventil, das am proximalen Ende, also am patientenfernen Ende des größeren Lumens des Tubus angeschlossen ist. Das Exspirationsventil, die Atemgasquelle, ein Druckminderventil in der Atemgaszufuhr und ein Tubus bilden ein Beatmungsgerät.

In Weiterbildung der Erfindung wird eine Beatmungsanlage unter Einsatz des erfindungsgemäßen doppellumigen Tubus gemäß Anspruch 22 vorgeschlagen.

Die kontrollierte oder unterstützende Beatmung erfolgt in ihrer herkömmlichen Weise über das Respirationslumen, das ist das größere Lumen des Tubus. Zur Verbesserung der Ventilation, d.h. Steigerung der Kohlendioxidelimination durch Reduktion des Totraumes, kann über das Inspirationslumen des Tubus zusätzlich ein kontinuierlicher, vorzugsweise exspiratorischer Atemgasfluß gegeben werden. Prinzipiell kann man kontinuierlich Luft oder Sauerstoff mit der Beatmungsanlage in das kleinere Lumen des Tubus einspeisen. Dann werden außer dem doppellumigen Tubus nur ein Durchflußmesser für Druckluft oder Sauerstoff als Atemgas aus der Atemgasquelle benötigt.

Für die Beatmung mit einem kontinuierlichen retrograden Atemgasfluß wird erfindungsgemäß für das steuerbare Exspirationsventil, das dem proximalen Ausgang des Respirationslumens des Tubus zugeordnet ist, ein elektrisches Steuergerät vorgesehen. Das Steuergerät arbeitet mit elektrischen Signalen, die von der Beatmungsanlage geliefert werden und öffnet das Exspirationsventil für den kontinuierlichen retrograden Atemgasfluß während der Ausatmungsphase, d.h. immer dann, wenn sich das Exspirationsventil in der Stellung "offen" befindet.

Natürlich kann die inspiratorische Sauerstoffkonzentration des kontinuierlichen oder exspiratorischen retrograden Atemgasflusses auch identisch sein mit der inspiratorischen Sauerstoffkonzentration, die die Beatmungsanlage bzw. das Beatmungsgerät, d.h. durch die Atemgasquelle abgegeben wird. Dies wird erreicht, indem der Durchflußmesser für den kontinuierlichen oder exspiratorischen retrograden Atemgasfluß an den Luft-/Sauerstoff-Mischer der Atemgasquelle oder an einen separaten Luft-/Sauerstoff-Mischer mit derselben Einstellung angeschlossen wird. Das Atemgas für den kontinuierlichen oder exspiratorischen retrograden Atemgasfluß kann in derselben Weise erwärmt und befeuchtet werden, wie dies bei dem von einem Beatmungsgerät abgegebenen Atemgas geschieht.

Insbesondere ist die Beatmung eines Patienten mit einer Beatmungsanlage unter Einsatz des erfindungsgemäßen doppellumigen Tubus mit einem kontinuierlichen retrograden Atemgasfluß möglich. Wenn zur Unterstützung der Beatmung mit einem kontinuierlichen retrograden Atemgasfluß in der Höhe eines Bruchteiles des erforderlichen Atemminutenvolumens begonnen wird und dann der kontinuierliche retrograde Atemgasfluß unter gleichzeitiger Zurücknahme der konventionellen Beatmung bis zum Mehrfachen des ursprünglich für die konventionelle Beatmung erforderlichen Atemminutenvolumens gesteigert wird, wird schließlich ein Punkt erreicht, an dem auf die konventionelle Beatmung über das Respirationslumen des Tubus völlig verzichtet werden kann. Alles Atemgas strömt dann über das Inspirationslumen des Tubus in die Luftröhre und verteilt sich bei geschlossenem Exspirationsventil in der Lunge - Einatmungsphase - oder entweicht bei offenen Exspirationsventil zusammen mit dem Gas, das während der Einatmungsphase in die Lunge strömte, über das Respirationslumen des Tubus nach außen - Ausatmungsphase. Diese Art der Beatmung wird als Beatmung mit einem kontinuierlichen retrograden Atemgasfluß bezeichnet.

In ihrer einfachsten Form kann die Beatmung mit einem kontinuierlichen retrograden Atemgasfluß manuell durchgeführt werden. Dann wird am kleineren Lumen des Tubus die Atemgasquelle angeschlossen (Atemgasfluß zum Beispiel 10 l/min.) und die beatmende Person verschließt manuell die äußere Öffnung des Respirationslumens des Tubus (Einatmungsphase) zum Beispiel für 3 Sekunden und gibt sie danach wieder frei (Ausatmungsphase), zum Beispiel ebenfalls für 3 Sekunden, so daß sich in diesem Beispiel ein Atemminutenvolumen von 5 l ergibt.

Prinzipiell kann für die Beatmung mit einem kontinuierlichen retrograden Atemgasfluß auch jedes handelsübliche Beatmungsgerät mit einem zeitgesteuerten Exspirationsventil und Atemgasquelle verwendet werden. Die Gasanschlüsse des Beatmungsgerätes bleiben dabei unbenutzt, das Gerät ist lediglich mit Strom versorgt. Hierbei wird das Inspirationslumen des Tubus mit der Atemgasquelle und das Respirationslumen des Tubus über einen Verbindungsschlauch mit dem Exspirationsventil verbunden. Am Beatmungsgerät können nun das Inspirations-/Exspirations-Verhältnis und die Atemfrequenz oder direkt die Inspirations- und Exspirationszeit eingestellt werden. Die Höhe des erforderlichen kontinuierlichen retrograden Atemgasflusses für eine effiziente Kohlendioxidelimination hängt dann von der Atemfrequenz und vom Inspirations-/Exspirations-Verhältnis ab. Der für die Oxygenierung entscheidende mittlere Atemwegsdruck wird über Veränderung des Inspirations-/Exspiration-Verhältnisses und das PEEP-Ventil bzw. die PEEP-Funktion des Beatmungsgerätes eingestellt. Hierbei ist Voraussetzung, daß ein entsprechendes zeitgesteuertes Exspirationsventil vorhanden ist. Die Aufgabe eines zeitgesteuerten Exspirationsventiles mit positiver Endausatmungsdruck-Funktion kann auch von einem von einem Beatmungsgerät unabhängigen Exspirationsventil geleistet werden, das für den Fall, daß bei einem Patienten ausschließlich die Beatmung mit einem kontinuierlichen retrograden Atemgasfluß durchgeführt wird, einsetzbar ist.

Der erfindungsgemäße doppellumige Tubus und das Prinzip der Beatmung mit einem kontinuierlichen retrograden Atemgasfluß mit einer Beatmungsanlage vermindern den Totraum bei der Beatmung eines Patienten derart, daß mit wesentlich höheren als den üblicherweise verwendeten Atemfrequenzen beatmet werden kann. Damit wird es erfindungsgemäß möglich, bei Durchführung einer Beatmung mit einem kontinuierlichen retrograden Atemgasfluß und Einsatz des erfindungsgemäß ausgebildeten Tubus Atemfrequenzen anzuwenden, die der menschlichen Herzfrequenz entsprechen. Erfindungsgemäß wird daher der doppellumige Tubus in einer Beatmungsanlage eingesetzt, die es ermöglicht, die Beatmung mit einem kontinuierlichen retrograden Atemgasfluß EKG - getriggert - und pulssychron durchzuführen.

Hierzu wird erfindungsgemäß eine Beatmungsanlage unter Einsatz des doppellumigen erfindungsgemäßen Tubus gemäß Anspruch 24 vorgeschlagen. Erfindungsgemäß wird das elektrische Steuergerät mit den Ausgangssignalen handelsüblicher Elektrokardiographen oder Blutdruckmeßgeräte beaufschlagt, um das Exspirationsventil, wie es in herkömmlichen Beatmungsgeräten oder Beatmungsanlagen oder als Gerät für sich vorhanden ist, pulssynchron zu schließen und zu öffnen.

Die Einatmungsphase kann mit einer erfindungsgemäß ausgerüsteten Beatmungsanlage mit der Systole synchronisiert werden. Diese Maßnahme ist in der Lage, die Nachlast der linken Herzkammer zu senken, was bei Links-Herzinsuffizienz (z.B. Herzinfarkt) hämodynamisch günstig ist. Es ist ebenfalls möglich, die Einatmungsphase mit der Diastole zu synchronisieren, was zur Folge hat, daß die rechte Herzkammer das Blut nicht gegen einen hohen Gefäßwiderstand in die vom Beatmungsdruck komprimierte Lungenstrombahn auswerfen muß. Die Maßnahme entlastet die rechte Herzkammer, was sich bei allen Zuständen mit akut erhöhtem pulmonalem Gefäßwiderstand, z.B. Lungenembolie, vorteilhaft auswirkt.

Vorteilhafte Weiterbildungen der Beatmungsanlage unter Einsatz des erfindungsgemäßen doppellumigen Tubus und eines elektrischen Steuergerätes für das Exspirationsventil sind den kennzeichnenden Merkmalen der Ansprüche 25 bis 36 entnehmbar.

Die Integration eines Elektrokardiographen in die Beatmungsanlage ermöglicht, mit den Signalen des Elektrokardiographen das Exspirationsventil, das an das größere Lumen des Tubus angeschlossen ist, so zu steuern, daß die Beatmung mit einem über das Inspirationslumen des Tubus zugeführten Atemgas pulssynchron erfolgt, eine sogenannte Herzschlag-synchrone Überdruckbeatmung. Das Steuergerät für das Exspirationsventil ist des weiteren mit Funktionen für die Veränderung des zeitlichen Verhältnisses von Einatmung zu Ausatmung (Inspirations-/Exspirations-Verhältnis) sowie für die alternative Anwahl einer Synchronisierung der Einatmung mit der Kontraktion des Herzmuskels (systolische Inspiration) oder mit seiner Erschlaffung (diastolische Inspiration) ausgerüstet.

In einer weiteren Ausbildung der Erfindung ist ein elektrisch steuerbares Ventil, insbesondere ein Druckminderungsventil vorgesehen, das in der Zufuhrleitung von der Atemgasquelle zu dem Inspirationslumen des Tubus angeordnet ist, und mit dessen Hilfe das zu dem Inspirationslumen des Tubus strömende Atemgas dosiert werden kann, so daß der Atemgasfluß während der Einatmung und der Atemgasfluß wahrend der Ausatmung frei wählbar sind.

Bei der Beatmung mit einem kontinuierlichen retrograden Atemgasfluß kann der Einfachheit halber ein konstanter Atemgasfluß eingestellt werden. Vorteilhafter ist jedoch die Möglichkeit, die Größe des kontinuierlichen Atemgasflusses für die Inspirationsphase und für die Exspirationsphase getrennt zu regeln. Hierfür ist das elektrisch steuerbare Druckminderventil vorgesehen. Der inspiratorische Atemgasfluß kann so an das für die Kohlendioxidelimination erforderlich Atemminutenvolumen angepaßt werden und der exspiratorische Atemgasfluß an den für die Oxygenierung maßgeblichen mittleren Atemwegsdruck.

Die Ausbildung des erfindungsgemäßen Tubus sowie eine erfindungsgemäße Anlage zur pulssychronen Beatmung mit einem retrograden Atemgasfluß wird nachfolgend an Ausführungsbeispielen dargestellt und erläutert. Es zeigen
- Figur 1: schematische Darstellung einer Beatmungsanlage
- Figur 2: Ansicht eines doppellumigen Tubus mit Düse
- Figur 3: den Teilquerschnitt DD nach Figur 6 des distalen Endbereiches des Tubus
- Figur 4: die Ansicht EE nach Figur 2
- Figur 5: den Querschnitt FF nach Figur 2
- Figur 6: die Draufsicht auf den Endbereich des Tubus nach Figur 2
- Figur 7: einen Düsenformkörper zum Einsetzen in das Inspirationslumen des Tubus nach Figur 2 in Draufsicht
- Figur 8: den Querschnitt GG des Düsenformkörpers nach Fig.7
- Figur 9: die Seitenansicht des Düsenformkörpers nach Fig. 7
- Figur 10: die Ansicht Y des Düsenformkörpers nach Figur 7
- Figur 11: auszugsweisen Längsschnitt des distalen Endbereiches des Tubus mit eingesetztem Düsenformkörper gemäß Fig. 7 bis 10
- Figur 12: auszugsweisen Längsschnitt durch den distalen Endbereich des Tubus mit Einsatz eines variierten Düsenformkörpers und Wulstausbildung
- Figur 13: die Schnittansicht analog dem Schnitt EE von Figur 2 des Tubusendes gemäß Figur 12
- Figur 14: den Querschnitt durch den Tubus gemäß Figur 12 entsprechend der Figur 5
- Figur 15: Ansicht eines doppellumigen Tubus in Doppelrohrausbildung
- Figur 16: Querschnitt KK durch den Tubus nach Figur 15
- Figur 17: Ansicht eines doppellumigen Tubus mit ringförmiger angesetzter Düse am distalen Ende des Tubus
- Figur 18: auszugsweiser Längsschnitt durch den distalen Endbereich des Tubus nach Figur 17 gemäß Schnitt MM von Figur 19
- Figur 19: Ansicht I des distalen Endes des Tubus nach Fig.17
- Figur 20: Querschnitt LL des Tubus nach Figur 17
- Figur 21: Blockdiagramm eines Steuergerätes für die Beatmungsanlage nach Figur 1
- Figur 22: Funktionsschema Steuergerät mit Beatmungsgerät
- Figur 23: Diagramm für die Ansteuerung des Exspirationsventils mittels Signale eines Elektrokardiographen
- Figur 24: Aufbau und Funktionsschema des Steuergerätes für eine Ansteuerung gemäß Figur 23
- Figur 25: Funktionsschema eines Steuergerätes mit integriertem Exspirationsventil für eine Ansteuerung gemäß Figur 23
- Figur 26: schematischer Aufbau eines Steuergerätes für das Exspirationsventil mit integriertem Taktgenerator.

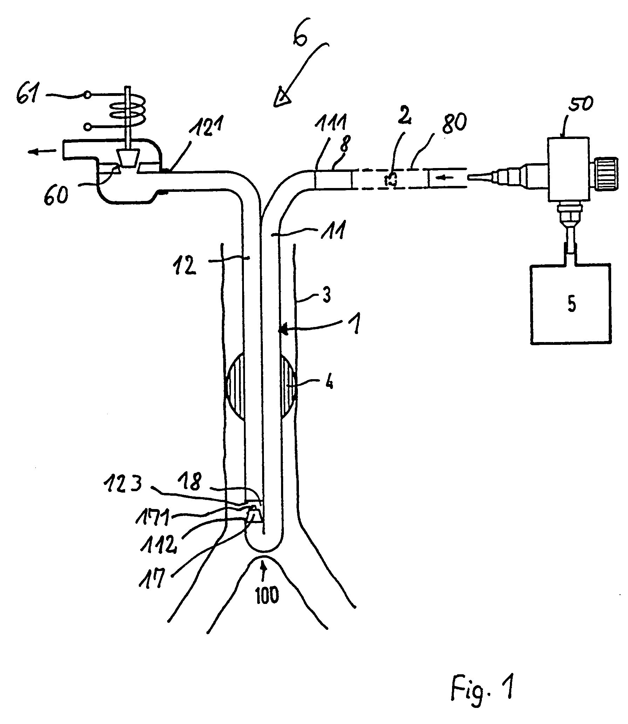

In der Figur 1 ist schematisch ein Beatmungsgerät 6 mit doppellumigen Tubus 1, Atemgasquelle 5 mit Durchflußmesser 50 und einem steuerbaren Druckminderventil 2 in der Atemgaszuleitung 80 zum Tubus 1 und einem elektrisch steuerbaren Exspirationsventil 60 mit elektrischen Anschlüssen 61 dargestellt. Der in die Luftröhre 3 eines Patienten bis zur Carina 100 eingeführte Tubus 1 besitzt zwei durchgehende Lumen 11, 12, von denen das als Inspirationslumen benutzte Lumen 11 einen kleineren Querschnitt als das als Respirationslumen benutzte Lumen 12 aufweist. Die gezeigte Beatmungsanlage 6 ist zur steuerbaren maschinellen Beatmung eines Patienten geeignet. Der doppellumige Tubus gemäß Figur 1 weist eine aufblasbare Manschette 4 zur Halterung und Abdichtung in der Luftröhre 3 auf. Das Inspirationslumen 11 ist eingangsseitig, d.h. am proximalen Ende 11 über einen Konnektor 8 und über den Durchflußmesser 50 an die Atemgasquelle 5 angeschlossen. Das Atemgas steht unter einem gewünschten Überdruck. Beatmungsgeräte mit nicht erfindungsgemäßen Tuben sind bekannt.

Das Respirationslumen 12 ist ausgangsseitig, d.h. am proximalen Ende 121 mit dem elektrisch steuerbaren Exspirationsventil 60 verbunden.

Im Bereich des distalen Endes des Tubus 1 in der Nähe der Carina 100 sind das Inspirationslumen 11 und das Exspirationslumen 12 über eine Düse 17, die am distalen Ende 112 des Inspirationslumens ausgebildet ist, miteinander gekoppelt. Die konische Düse 17 liegt mit ihrer Düsenaustrittsöffnung 171 bzw. Düsenmund nahe bzw. an der Austrittsöffnung 123 des Respirationslumens 12, so daß zwischen dem Düsenaustritt und dem distalen Ende des Respirationslumens 12 bzw. der Innenwandung des Respirationslumens 127 ein offener Ringraum 18 verbleibt. Wird das Exspirationsventil 60 geschlossen, füllt das unter Druck stehende Atemgas über das Inspirationslumen 11 des Tubus 1, austretend an der Düse 17, die Lunge. Bei geöffnetem Exspirationsventil 60 strömt das Atemgas über die Düse 17 in das Respirationslumen 12 vom distalen Ende her ein und durch das Respirationslumen 12 und das Exspirationsventil 60 nach außen ab. Bei diesem Vorgang wird zugleich das Gas in der Lunge durch den im freien Ringraum 18 entstehenden Unterdruck über das Respirationslumen 12 und das Exspirationsventil 60 nach außen abgeführt. Die Düse 17 am distalen Ende des Inspirationslumens 11 ist so angeordnet, daß sie auf das distale Ende des Respirationslumens gerichtet ist und aus der Düse des Inspirationslumens in das Respirationslumen hineinbläst, und zwar in Richtung der Ausatmung.

Zwischen der Atemgasquelle 5 und dem Inspirationslumen 11 des Tubus 1 kann ein gestrichelt dargestelltes elektrisch steuerbares Druckminderventil 2 eingesetzt werden, das ermöglicht, für die Einatmungsphase und die Ausatmungsphase verschiedene Atemgasdrücke vorzusehen. Hierdurch kann zum Beispiel der Ausatmungsvorgang zeitlich beschleunigt werden.

Die weitere erfindungsgemäße Ausgestaltung der Beatmungsanlage 6 nach Figur 1 zur steuerbaren maschinellen Beatmung besteht darin, das Exspirationsventil 60 in Abhängigkeit von einem Signal eines Elektrokardiographen zu steuern. Die derart ausgerüstete Anlage ermöglicht dann, eine Beatmung mit positivem Druck synchron zum Herzzyklus durchzuführen. Beispielsweise kann die Effizienz des insuffizienten linken Ventrikels durch eine systolische Inspiration, sogenannte Nachlastsenkung, und eine diastolische Exspiration, sogenannte Minimierung der Vorlastsenkung, verbessert werden. Ebenso kann in Situationen mit akuter Rechts-Herzinsuffiezenz der rechte Ventrikel mittels einer diastolischen Inspiration und einer systolischen Exspiration entlastet werden.

Je nach therapeutischer Zielsetzung können das elektrisch steuerbare Exspirationsventil 60 und/oder das elektrisch steuerbare Druckminderventil 2 von Signalen gesteuert werden, die ihrerseits von medizinischen Geräten, wie zum Beispiel einem Elektrokardiographen, oder aber von einem diese Geräte ersetzenden Taktgenerator gesteuert werden.

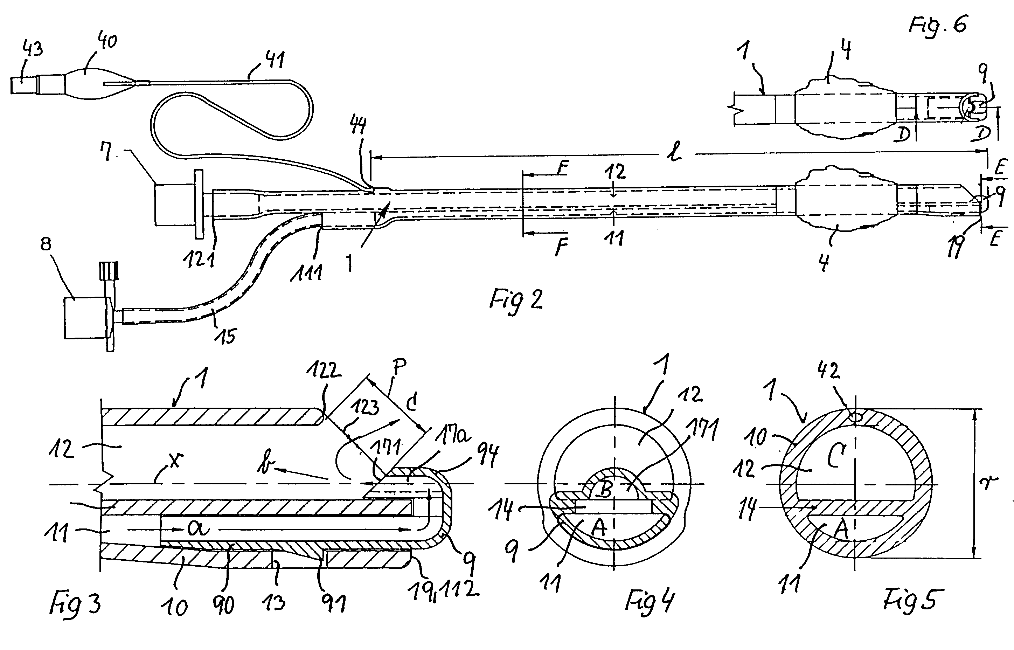

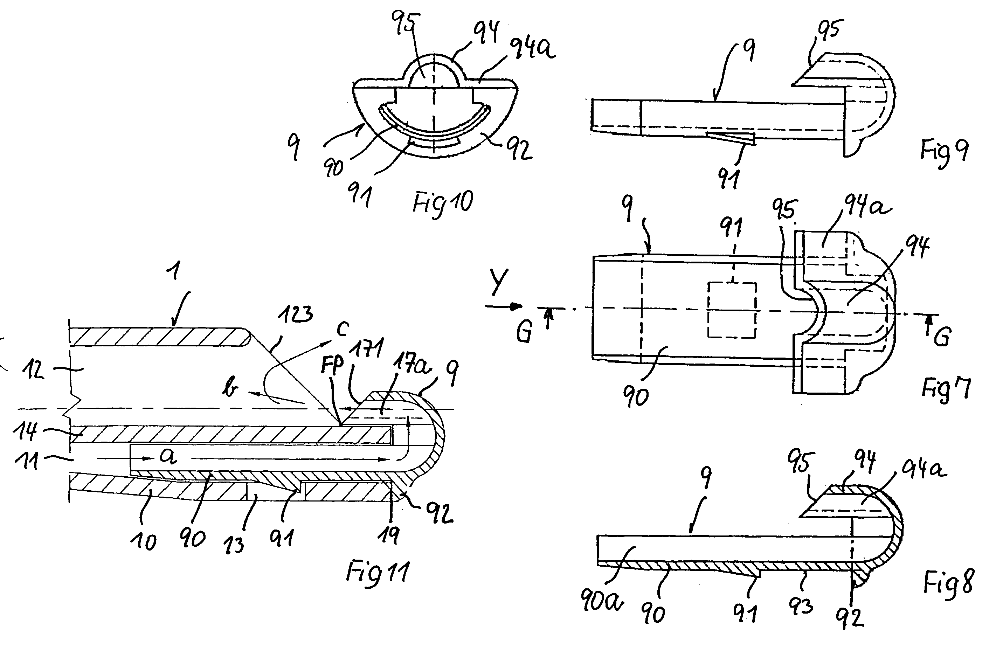

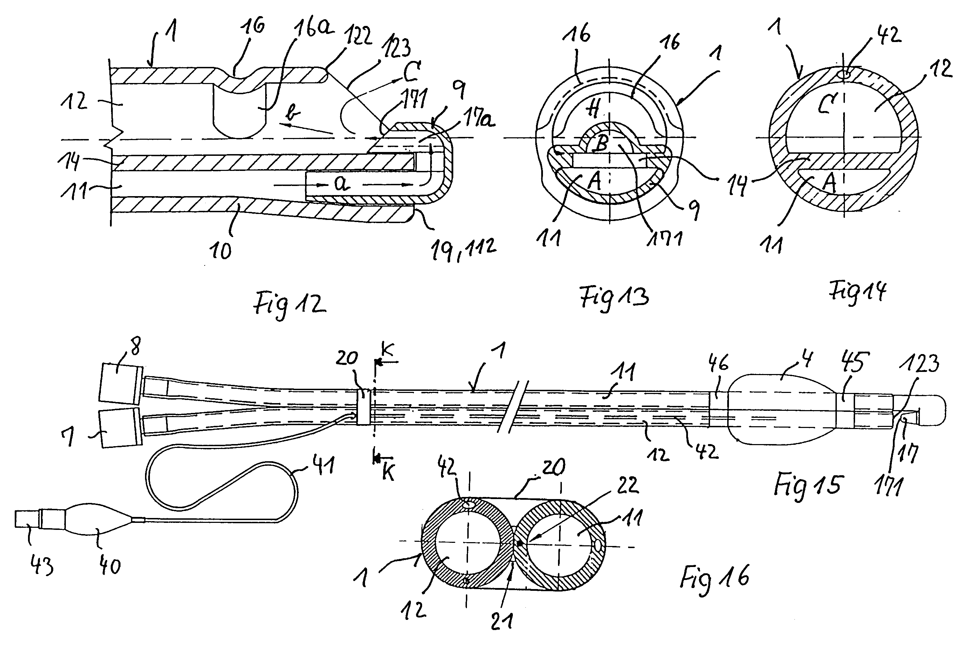

In den Figuren 2 bis 6 ist ein doppellumiger Tubus 1, der geeignet ist zum Einsatz bei einer steuerbaren maschinellen Beatmung mit einem kontinuierlichen retrograden Atemgasfluß, dargestellt. Der Tubus 1 weist zwei durchgehende Lumen 11, 12 auf, die durch eine Trennwand 14, siehe Figur 3, voneinander abgeteilt sind. Die durchgehenden Lumen weisen unterschiedliche Größe auf, wobei das größere Lumen als Respirationslumen für die ungehinderte konventionelle Beatmung als auch eine Spontanatmung des Patienten dient, des weiteren im Querschnitt so groß sein soll, daß die Einführung eines Absaugkatheters oder eines Bronchoskops möglich ist. Das kleinere Lumen 11 dient als Inspirationslumen der Atemgaszufuhr. Der Tubus 1 ist so lang, daß unabhängig von der Tubus- bzw. Patientengröße ein Teil des Tubusschaftes aus dem Patienten ragt und zur Befestigung, vorzugsweise mit Klebe- oder Klettband geeignet ist. Die Länge 1 eines durchschnittlichen Tubus beträgt beispielsweise 220 mm. Im Bereich des distalen Endes 19 des Tubus mit etwas Abstand von demselben ist eine aufblasbare Tubusmanschette 4 vorgesehen. Die Tubusmanschette 4 wird über einen Luftzuführungskanal 42, der in der Wandung des Tubus 10 ausgebildet ist, mit Blasluft versorgt, siehe Figur 5. Im Bereich des proximalen Endes des Tubus 1 ist der Kontrollballon 40 mit Anschluß 43 über den Luftzuführungsschlauch 41 am Ausgang 44 des Luftkanals 42 angebracht. Am proximalen Ende 121 des größeren Lumens 12 ist ein passender Anschlußstutzen 7 vorgesehen. Am proximalen Ausgang 111 des Inspirationslumens 11 ist ebenfalls ein Anschlußstutzen 8 vorgesehen, der für die bessere Handhabbarkeit über einen Zufuhrschlauch 15 mit dem proximalen Eingang des Inspirationslumens verbunden ist. Am distalen Ende 19 des Tubus 1 ist das Inspirationslumen 11 düsenförmig verengt ausgebildet, siehe Figur 3 und 4, wobei der Düsenkanal der Düse 17 um 180° abgewinkelt verläuft, so daß die Düsenaustrittsöffnung 171 auf das offene distale Ende 122 des Respirationslumens 12 weist. Der durch das Inspirationslumen 11, siehe Figur 3, in Pfeilrichtung a einströmende Atemgasfluß wird am distalen Ende des Tubus 19 durch die Ausbildung der Düse 17 mit sich gegenüber dem Querschnitt des Inspirationslumens 11 zum Düsenaustritt hin verkleinerndem Querschnitt bei gleichzeitiger Umlenkung um 180° in Richtung auf das Respirationslumen 12 geführt unter gleichzeitiger Beschleunigung und strömt in Pfeilrichtung b, d.h. nunmehr in entgegengesetzter Richtung, vom distalen Ende des Tubus her in das Respirationslumen 12 ein. Für den Fall, daß das Exspirationsventil, siehe Figur 1, am proximalen Ausgang des Lumens 12 geschlossen ist, kann das in Pfeilrichtung b in das Respirationslumen einströmende Atemgas nach Füllung des Respirationslumens 12 durch die distale Austrittsöffnung 123 des Respirationslumens in Pfeilrichtung c austreten und in die Lunge gelangen. Bei Öffnung des Exspirationsventils am proximalen Ausgang des Respirationslumens 12 hingegen, reißt der in Pfeilrichtung b sich in das Respirationslumen 12 vom distalen Ende her bewegende Atemgasfluß das aus der Lunge kommende Ausatmungsgas mit sich und bewirkt eine gute Entleerung der Lunge von Ausatmungsgas und erhebliche Verbesserung der Ventilation, d.h. Steigerung der Kohlendioxidelimination durch Reduktion des Totraumes.

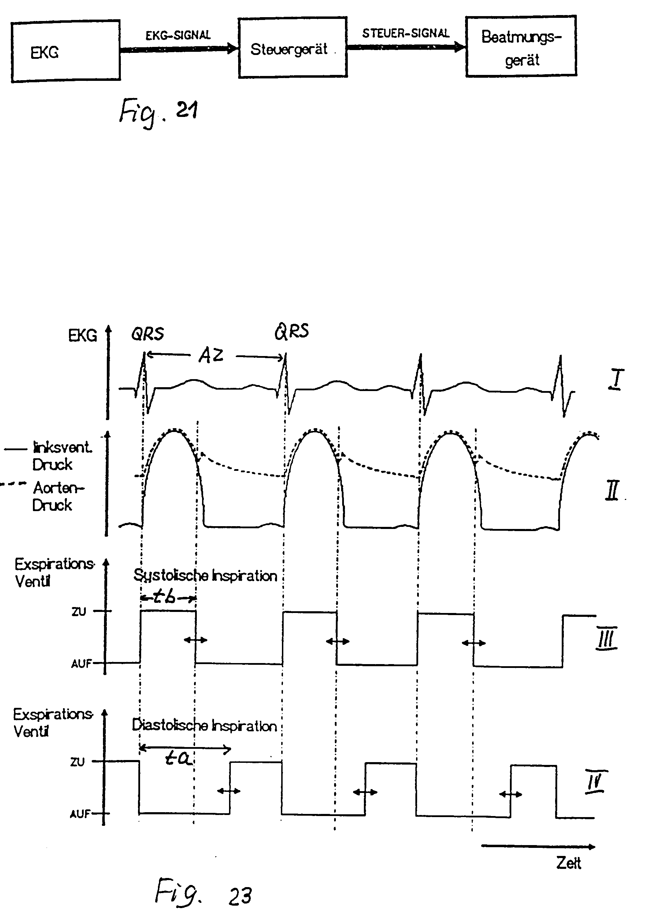

In dem gezeigten Beispiel gemäß Figuren 2 bis 6 ist die Ausbildung des distalen Endes 112 des Inspirationslumens 11 mit einer Düse unter Umlenkung des Strömungskanals mit Hilfe eines in das distale Ende des Tubus und das Inspirationslumen 11 eingesetzten Düsenformstückes 9 dargestellt. Das Düsenformstück 9 liegt mit einem Schenkel 90 innenseitig an der Wandung des Tubus 10 an, so daß zwischen der Trennwand 14 und dem Schenkel 90 ein Strömungskanal in Fortsetzung des Inspirationslumens 11 gebildet ist. Das Düsenformstück 9 ist in Verlängerung des Schenkels 90 hakenförmig mit einem kürzeren Schenkel 94 ausgebildet, der um das distale Ende der Trennwand mit Abstand unter Ausbildung des Düsenkanals 17a herumgeführt ist. Der Düsenkanal 17a endet mit einer Strömungsrichtung, die achsparallel zur Längsachse X des Tubus verläuft. Die Austrittsöffnung 171 des Düsenkanals und damit des Inspirationslumens 11 am distalen Ende ist in dem gezeigten Beispiel gemäß Figur 3 nach außen abgeschrägt ausgeführt, wodurch eine verbesserte Abströmung auch in Pfeilrichtung c bei Einatmung ermöglicht ist. Des weiteren ist die Düsenaustrittsöffnung 171 des Inspirationslumens 11 an der Peripherie des Querschnittes des Respirationslumens 12 angeordnet, so daß eine möglichst große Austrittsöffnung 123 für das Respirationslumen 12 verbleibt. Diese Austrittsöffnung 123 kann beispielsweise in dem gezeigten Beispiel einen Durchmesser p von 5,9 mm aufweisen. Die Tubuswandung 10 sollte möglichst gering sein, sie kann bei Auswahl eines geeigneten Werkstoffes, beispielsweise PVC, 1,22 mm betragen. Die Austrittsöffnung 123 muß auch so groß sein, daß sie das ungehinderte Hindurchführen eines Absaugkatheters durch das Respirationslumen 12 zum Absaugen der Atemwege ermöglicht. Aus diesem Grunde verläuft die Austrittsöffnung 123 auch abgeschrägt, beispielsweise unter 45° in bezug auf die Längsachse X des Tubus 1 dergestalt, daß die Tubusspitze einseitig zum distalen Ende 19 hin verjüngt ist. Auch auf diese Weise wird die Austrittsöffnung 123 vergrößert. Darüber hinaus ist es möglich, durch weiteres Auseinanderrücken von distaler Austrittsöffnung 123 des Respirationslumens 12 und distaler Austrittsöffnung 171 des Inspirationslumens 11 den Ausgangsbereich zu vergrößern, wozu beispielsweise auf das Ausführungsbeispiel gemäß Figur 11 verwiesen wird, wo die Austrittsöffnungen 123 und 171 einander lediglich im Fußpunktbereich FP an der Trennwand 14 berühren.

Das Düsenformstück 9 ist im Bereich der Tubuswandung 10 mechanisch mittels einer vorstehenden Rastnase 91, die in eine Ausnehmung 13 in der Tubuswandung eingreift, verrastet. Des weiteren ist das Tubusformstück 9 im Bereich der Tubuswandung 10 verklebt bzw. abgedichtet fixiert.

Für eine gute Funktion des doppellumigen Tubus, insbesondere eine gute Ventilation und Atemgasführung betreffend, ist die Größe der Querschnitte des Respirationslumens, des Inspirationslumens sowie der Düsenaustrittsöffnung für sich genommen und ihr Verhältnis zueinander maßgeblich. Der Querschnitt des Inspirationslumens muß ausreichend groß sein, um eine bis zum 5-fachen des spontanen Atemminutenvolumens ausreichende Atemgaszufuhr unter üblichen Drücken durchzulassen. Der Querschnitt des Respirationslumens muß die ungehinderte konventionelle Beatmung oder Spontanatmung des Patienten erlauben und die Hindurchführung eines Absaugkatheters. Unter diesen Umständen ist ein Verhältnis des Querschnittes C des Respirationslumens zu dem Querschnitt A des Inspirationslumens zu der Größe B der Düsenaustrittsöffnung 171 des Inspirationslumens von etwa 8:2:1 vorgesehen. Die Querschnitte A, B, C, wie vorangehend aufgeführt, sind aus den Schnittdarstellungen gemäß Figur 4 und 5 für den Katheter gemäß Figur 2 ersichtlich. Bei einem Innendurchmesser des Respirationslumens 12 entsprechend einem konventionellen Tubus mit 7 mm Innendurchmesser beträgt die Querschnittsfläche C ca. 38,5 mm², die Querschnittfläche A ca. 9,6 mm² und die Düsenaustrittsfläche B ca. 4,8 mm². Entsprechend wäre der Außendurchmesser r des Tubus 1 etwa 11 mm.

Als Konnektoren für das Inspirationslumen 11 können bevorzugt solche mit integriertem Abnahmestutzen zum Monitoring des Atemwegsdruckes oder der Atemgaszusammensetzung eingesetzt werden, gegebenenfalls auch für den proximalen Eingang des Respirationslumens 12.

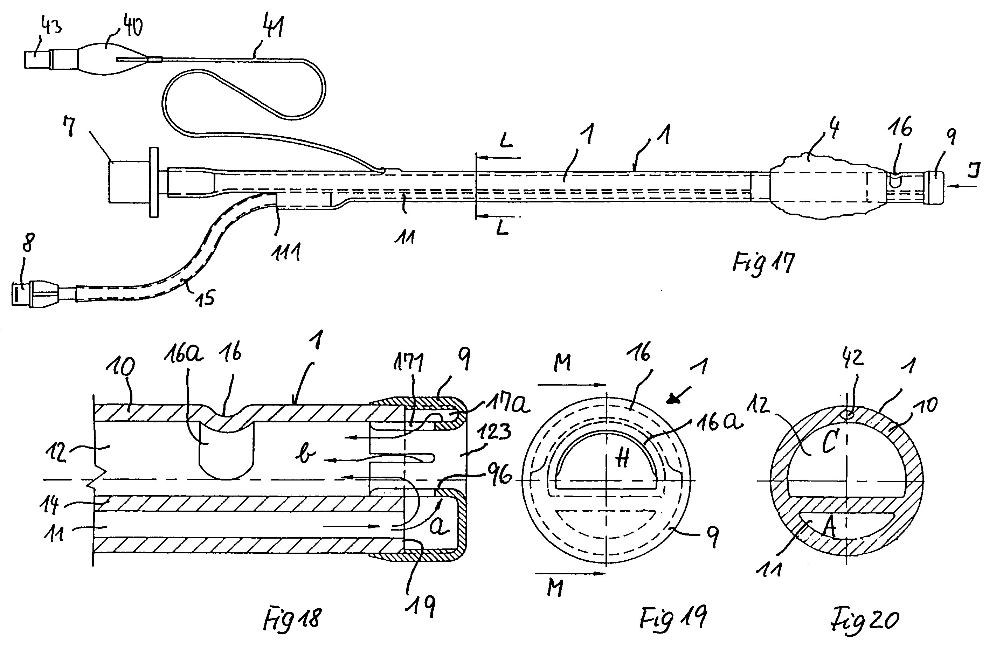

In den Figuren 7 bis 10 ist eine weitere Variante eines Düsenformstückes 9 zum Einsetzen in das Inspirationslumen 11 zur Ausbildung einer Düsenaustrittsöffnung am distalen Ende des Tubus 1 dargestellt. Das Düsenformstück weist ebenfalls einen langen Schenkel 90 zum Einführen in das Inspirationslumen 11 vom distalen Ende 19 des Tubus her auf, wie in der Figur 11 ersichtlich, wobei die Tubuswandung 10 entsprechend der Dicke des Schenkels 90 nach außen aufgeweitet ist, um das Inspirationslumen 11 mit gleichem Querschnitt eingangs des Düsenformstückes fortzusetzen. Das Düsenformstück weist des weiteren den abgewinkelten um 90° führenden Schenkel 94 auf, der zu einer Umlenkung des Strömungskanals um 180° führt und am Ende zur Austrittsöffnung mit einer Schräge 95 endet. Der von dem kurzen Schenkel 94 eingeschlossene Kanal ist durch zusätzliche Abwinkelungen 94a querschnittsmäßig zum Ende 95 hin sich verjüngend und verengend ausgebildet. In Verbindung mit der Trennwand 14 wird dann der geschlossene Strömungskanal für das Atemgas in Pfeilrichtung a gebildet, siehe Figur 11. Zur Verankerung des Düsenformstückes 9 am Tubus 1 sind außenseitig am längeren Schenkel der vorstehende Rasthaken 91 und die Anschlagnase 92 mit Aufnahmetasche 93 für die Verrastung, siehe Figur 11, an der Tubuswandung 10 gebildet. Je nach Zuordnung der von dem am Tubusende eingesetzten Düsenformstück 9 gemäß Figur 11 gebildeten Düsenaustrittsöffnung 171 für das Inspirationslumen 11 und der Ausbildung der Düsenaustrittsöffnung 123 des Respirationslumens 12 wird eine entsprechende Ventilation, angedeutet durch die Pfeile c und b für das durch das Inspirationslumen 11 zugeführte Atemgas ermöglicht.

In der Figur 12 ist eine weitere Variante eines Düsenformstückes 9 zur Ausbildung der düsenförmigen Verengung am distalen Ende des Inspirationslumens und Düsenaustrittsöffnung 171, eingesetzt am distalen Ende 19 des Tubus 1, dargestellt. Hierbei ist lediglich ein relativ kurzes Düsenformstück mit seinem langen Schenkel 9 in das ausgeweitete Ende des Tubus in das Inspirationslumen 11 eingesteckt und mit diesem verklebt.

Zur Erzielung eines Venturi-Effektes, der die aus den Atemwegen durch den Atemgasstrom b mitgerissene Gasmenge erhöhen soll, kann nahe der distalen Austrittsöffnung 123 des Respirationslumens 12 ein Wulst 16a in das Respirationslumen zur Querschnittsverringerung mittels einer Kerbe 16 in die Wandung 10 des Tubus eingedrückt sein. Hierbei kann eine Querschnittsverringerung bis zu etwa 25 % vorgesehen werden.

Figur 13 zeigt die Ansicht auf das distale Ende 19 des Tubus im Querschnitt, wobei mit H die durch den Wulst 16a verringerte Querschnittsfläche des Respirationslumens 12 bezeichnet ist.

In der Figur 15 ist eine Ausbildung eines doppellumigen Tubus mit zwei zu einem Tubus zusammengeführten Rohren, enthaltend das kleinere Inspirationslumen 11 und das größere Respirationslumen 12, dargestellt. Die beiden die Lumen enthaltenden Rohre sind mittels Bänder 20 und Bänder 45, 46 im Bereich der aufblasbaren Tubusmanschette 4 miteinander gekoppelt. Zusätzlich können die den Tubus 1 bildenden Rohre, siehe Querschnitt Figur 16, im Berührungsbereich 22 mit Klebemitteln fest miteinander verbunden sein. Es ist auch möglich, diese in diesem Bereich entsprechend bei Einsatz eines geeigneten Kunststoffes miteinander zu verschweißen, beispielsweise mittels Lösungsmitteln. Bei dem Tubus 1 gemäß Figur 15 ist die Austrittsöffnung 171 des düsenförmigen um 180° umgelenkten Endes des Inspirationslumens 11 mit etwas Abstand von der distalen Austrittsöffnung 123 des Respirationslumens 12 angeordnet, so daß einerseits ein freier Abfluß des aus der Düsenaustrittsöffnung 171 austretenden Atemgases bei geschlossenem Exspirationsventil in die Lungen erfolgen kann, zum anderen die distale Austrittsöffnung 123 zum Durchstecken eines Absaugkatheters ausreicht und des weiteren bei geöffnetem Exspirationsventil der Atemgasstrom aus der Düsenaustrittsöffnung 171 direkt in die Austrittsöffnung 123 in das große Respirationslumen 12 hineinblasen kann.

In der Figur 17 ist eine weitere Ausgestaltung des doppellumigen Tubus 1 gemäß der Erfindung dargestellt, bei dem das distale Ende des Tubus 1 mit einem ringförmig gestalteten Düsenformstück versehen ist, das sowohl den sich verengenden Düsenaustrittskanal am distalen Ende für das Inspirationslumen 11 bildet, als auch die Austrittsöffnung 123 des Respirationslumens 12 am distalen Ende formt.

Figur 19 zeigt hierbei die Ansicht I gemäß Figur 17 auf das distale Ende des Tubus mit Düsenformstück 9. Der axiale Längsschnitt MM durch Figur 19 zeigt in der Figur 18 den distalen Endbereich des Tubus im Querschnitt und das ringförmig ausgebildete Formstück 9, das außenseitig an der Tubuswandung 10 übergreifend fixiert ist und innenseitig einen sich verengenden Ringraum mit mehreren Düsenaustrittsöffnungen 171 bildet. Die innere Düsenringwand 96 bildet zugleich die Begrenzung für die Austrittsöffnung 123 des Respirationslumens. Mit etwas Abstand vom distalen Ende 19 des Tubus 1 ist wiederum im Bereich des Respirationslumens 12 eine Einkerbung 16 zur Ausbildung einer den Querschnitt teilweise und über eine kurze Strecke verkleinernden Wulst 16a ausgebildet.

Figur 20 zeigt wiederum, daß der Tubus im mittleren Bereich, siehe Querschnitt LL gemäß Figur 17 das durchgehende Respirationslumen 12 mit Querschnitt C und das durchgehende Inspirationslumen 11 mit Querschnitt A aufweist mit einer ausreichenden Größe zur Erfüllung ihrer Funktionen.

Für eine pulssynchrone Beatmung mit einem kontinuierlichen retrograden Atemgasfluß wird das Exspirationsventil 60 des Beatmungsgerätes 6 gemäß Figur 1 mit Hilfe eines Steuergerätes angesteuert. Für die pulssynchrone Beatmung ist das Steuergerät mit einem Elektrokardiographen EKG verbunden, dessen EKG-Signal vom Steuergerät empfangen, elektronisch weiterverarbeitet und als Steuersignal für das Exspirationsventil benutzt wird.

Figur 21 zeigt im Blockdiagramm das Funktionsschema. Das Steuergerät stellt eine Verbindung zwischen EKG oder Blutdruckmeßgerät und dem Exspirationsventil eines Beatmungsgerätes her und liefert das Steuersignal um das Exspirationsventil zu öffnen bzw. zu schließen. Das Grundsystem einer Beatmungsanlage für eine pulssynchrone Beatmung ist schematisch in der Figur 22 dargestellt und umfaßt die folgenden Teile: eine Atemgasquelle, einen doppellumigen Tubus mit Düse am distalen Ende des Inspirationslumens, ein Exspirationsventil, ein Steuergerät und einen Elektrokardiographen oder Blutdruckmeßgerät. Die Beatmungsanlage kann mit handelsüblichen EKG-Geräten oder Blutdruckmeßgeräten sowie handelsüblichen Beatmungsgeräten mit Exspirationsventil, beispielsweise einem Servo-Ventilator 900C der Firma Siemens in Verbindung mit dem erfindungsgemäßen elektronischen anpaßbaren Steuergerät und in Verbindung mit dem erfindungsgemäßen doppellumigen Tubus mit Düsenaustritt am distalen Ende des Inspirationslumens ausgestattet werden und ermöglicht eine pulssynchrone Beatmung in der Anästhesie, Intensiv- und Notfallmedizin.

Die Zeitdiagramme für eine pulssynchrone Beatmung sind in Figur 23 dargestellt. Die Kurve I stellt die vom EKG-Gerät erhaltenen EKG-Signale mit den QRS-Komplexen dar. Die aus dem zeitlichen Abstand AZ zweier QRS-Komplexe voneinander berechnete Zeitdauer AZ entspricht der Zeitdauer eines Atemzyklus. Die Kurve II zeigt den Verlauf des Druckes in der Aorta und im linken Ventrikel. Die Kurve III zeigt die systolische Inspiration und die Kurve IV zeigt die diastolische Inspiration.

Um die pulssynchrone Beatmung durchzuführen, ist die Kontraktionszeit (Systole) des Herzens zu bestimmen. Der Anfang der Systole kann von dem QRS-Komplex des Elektrokardiographen abgeleitet werden. Für das Ende der Systole gibt es keine definierten technischen Signale. Deshalb wird folgende Abschätzung durchgeführt: Eine Herzperiode besteht aus drei Phasen, der Kontraktion, der Erschlaffung und der Ruheperiode, wobei unter normalen Herzaktivitäten jede dieser Phasen etwa ein Drittel der gesamten Periode zwischen zwei Herzschlägen einnimmt. Die Zeit für die Systole wird daher auf ein Drittel der Zeit zwischen zwei Herzschlägen, d.h. zwischen zwei aufeinanderfolgenden QRS-Komplexen, angenommen. Um eine definierte Synchronisation zu erhalten, kann das Verhältnis zwischen Inspiration und Exspiration variiert werden. Bei der systolischen Beatmung wird der Beginn der Inspiration bestimmt von dem QRS-Komplex des EKG-Signals. Die Dauer der Systole wird von der Pulsfrequenz bestimmt, die bestimmt wird durch das Intervall der jeweils vorangehenden QRS-Komplexe, ebenso wie von dem eingestellten Inspirations-/Exspirationsverhältnis. Mit dem Ende der Systole wird auf Ausatmung umgeschaltet und auf ein neues QRS-Signal, um eine neue Inspiration zu starten, gewartet.

Bei der diastolischen Inspiration wird die Exspiration mit einem QRS-Komplex in dem EKG-Signal gestartet. Von der Pulsfrequenz, die aus zwei vorangehenden QRS-Komplexen bestimmt wird, ebenso wie von dem I/E-Verhältnis wird der Zeitpunkt des Beginns der Inspiration bestimmt. Wenn dieser Punkt erreicht ist, wird auf Inspiration umgeschaltet. Danach wird wiederum auf den nächsten QRS-Komplex gewartet. Bei der systolischen Beatmung ist also der Beginn der Einatmungsphase synchron mit dem QRS-Komplex, während bei der diastolischen Beatmung das Ende der Inspiration synchron mit dem QRS-Komplex ist.

Während der Inspiration ist das Exspirationsventil geschlossen und das Atemgas strömt in die Lunge. Während der Exspiration ist das Exspirationsventil offen und die Luft wird aus der Lunge durch den Atemgasstrom mit nach außen gerissen.

Der funktionelle Aufbau des Steuergerätes zur Durchführung einer pulssynchronen Beatmung gemäß Figur 23 ist in der Figur 24 dargestellt. Das Steuergerät enthält eine Signalaufbereitung, eine Auswerte-Elektronik, eine Ansteuer-Elektronik sowie Anzeigeelemente und Bedienungselemente (Tastenerkennung). Die Signalaufbereitungs-Elektronik hat die Aufgabe, das von dem Elektrokardiographen oder Blutdruckmeßgerät gelieferte elektrische EKG-Signal zu empfangen, aufzuschlüsseln und den QRS-Komplex darzustellen, um dann einen definierten Steuerimpuls an die Auswerte-Elektronik zu geben. Die Signalaufbereitungs-Elektronik enthält einen Schwellenwertdetektor und ein Zeitglied, das den Steuerimpuls mit der nötigen Länge und Amplitude ausstattet. Dies ermöglicht, EKG-Geräte mit analogen oder digitalen Signalen zu benutzen. Damit wird es möglich, alle handelsüblichen EKG-Geräte oder Blutdruckmeßgeräte für die erfindungsgemäße Beatmungsanlage für eine pulssynchrone Beatmung einzusetzen.

Die Auswerte-Elektronik ist der zentrale Bestandteil des Steuergerätes. Die Auswerte-Elektronik enthält die folgenden funktionalen Einheiten: Zentrale Prozessoreinheit, Taktgeber, RAM-Speicher, Schnittstellen für Ein- und Ausgänge, Zeitglied, Unterbrechungs-Kontrollkreis und gegebenenfalls einen Festwertspeicher (ROM). Die Auswerte-Elektronik übernimmt den von der Signalaufbereitungs-Elektronik gelieferten Impuls und berechnet aus der Zeitdauer des Abstandes AZ zweier QRS-Komplexe die Dauer zwischen zwei Herzschlägen. Die Auswerte-Elektronik ist des weiteren mit den Bedienungselementen, mit denen das Inspirations-/Exspirations-Verhältnis sowie die Betriebsart bestimmbar sind, verbunden und es kontrolliert die Anzeigeelemente. In der Auswerte-Elektronik wird die Zeitdauer für die Inspiration und die Exspiration entsprechend dem eingestellten Inspirations-/Exspirationsverhältnis festgelegt sowie die Zeit zwischen den EKG-Signalen und die anzuwendende Betriebsart. Die Auswerte-Elektronik sendet das erforderliche Signal zum Öffnen und Schließen des Exspirationsventils. Die Ansteuerung des Exspirationsventiles erfolgt dann in Abhängigkeit von der Pulsfrequenz (EKG-Signale) und dem eingestellten vorgegebenen Inspirations-/Exspirations-Verhältnis (I/E-Verhältnis). Mit dem I/E-Verhältnis kann der Beatmungszyklus zur Systole bzw. Diastole synchronisiert werden. Im Unterschied zu einem EKG-Trigger mit einstellbaren, dann aber fixierter Verzögerung ist man mit dem erfindungsgemäßen Steuergerät und seiner Ansteuerung unabhängig von der Pulsfrequenz. Es ist möglich, mit einer frei wählbaren Frequenz zu beatmen. Das Verhältnis von Inspiration- zu Exspirationszeit ist einstellbar, beispielsweise in einem Bereich von 1:4 bis 4:1. Die Dauer von Ein- und Ausatmung wird durch das eingestellte I/E-Verhältnis bestimmt. Der richtige Zeitpunkt für die Umschaltung von Einauf Ausatmung, d.h. die Öffnung des Exspirationsverntil, resultiert aus der eingestellten Betriebsart (systolische oder diastolische Inspiration) und dem eingestellten I/E-Verhältnis.

Die Ansteuer-Elektronik übernimmt die notwendigen Anpassungen des von der Auswerte-Elektronik gelieferten Ansteuersignales, um die Exspirationsventile von handelsüblichen Beatmungsgeräten steuern zu können. Die Tastenerkennung des Steuergerätes ermöglicht, mittels Bedienungselementen das Verhältnis von Inspiration zu Exspiration einzustellen. Des weiteren sind Bedienungselemente zum Einstellen der Betriebsart vorhanden. Die Tastenerkennung wird in der Auswerte-Elektronik erfaßt und umgesetzt.

Das Steuergerät besitzt des weiteren Anzeigenelemente zur Anzeige der Betriebsart, des eingestellten I/E-Verhältnisses. Außerdem werden eingehende QRS-Komplexe (EKG-Signale) sowie die Stellung des Exspirationsventils angezeigt. Alle Anzeigeelemente werden durch die zentrale Auswerte-Elektronik des Steuergerätes angesteuert. Die Auswerte-Elektronik des Steuergerätes benötigt ein externes Programm.

Das Steuergerät unterscheidet zwei Betriebsarten.
- Betriebsart "systolisch": Die Einatmung erfolgt in der Systole des Herzens.
- Betriebsart "diastolisch": Die Einatmung erfolgt in der Diastole des Herzens.

Man kann zwischen beiden Betriebsarten mittels Bedienungselementen wechseln, hierfür ist die Auswerte-Elektronik über eine Tastenerkennung ansteuerbar, siehe Figur 24.

In der Betriebsart "diastolisch" wird sofort nach dem Erkennen eines EKG-Signales (QRS-Komplex) das Exspirationsventil geöffnet, siehe Figur 23, Kurve IV. Abhängig vom I/E-Verhältnis wird dann das Exspirationsventil nach einer bestimmten Zeit ta wieder geschlossen, so daß die Inspiration während der Diastole erfolgt. Nach Eintreffen des nächsten QRS-Komplexes wird das Exspirationsventil wieder geöffnet.

In der Betriebsart "systolisch" wird nach dem Erkennen des QRS-Komplexes das Exspirationsventil geschlossen, siehe Figur 23, Kurve III. In dieser Betriebsart wird abhängig vom I/E-Verhältnis das Exspirationsventil nach einer bestimmten Zeit tb wieder geöffnet, so daß die Inspiration während der Systole stattfindet. Nach dem Eintreffen des nächsten QRS-Komplexes wird das Exspirationventil wieder geschlossen.

Das Steuergerät überwacht die eigene Funktionalität selbst. Um ein Maximum an Sicherheit zu erreichen, sind die Überwachungsfunktionen der angeschlossenen Beatmungsanlage aktiv. Wird eine Fehlfunktion des Beatmungssystems erkannt, so wird das Steuergerät automatisch abgeschaltet und das konventionelle Beatmungsgerät beginnt selbständig mit der konventionellen Beatmung.

Die Auswerte-Elektronik des Steuergerätes prüft ständig die Zeiten für die Inspiration und Exspiration und das Ankommen neuer EKG-Signale. Nur wenn mehrere korrekte Zyklen der Beatmung festgestellt wurden, übernimmt das Steuergerät die Überwachung des Beatmungsgerätes. Dann wird der Abschalter zum Beatmungsgerät geschlossen und eine Kontrolleuchte zeigt dies an. Im Falle von Stromausfällen trennt der Abschalter das Steuergerät von dem Beatmungsgerät, das dann wieder selbständig zu arbeiten beginnt.

Im Falle des Fehlens von EKG-Signalen wird ebenfalls die Fortführung der konventionellen Beatmung mit dem Beatmungsgerät ohne Steuerung durch das Steuergerät veranlaßt, solange bis neue EKG-Impulse das Steuergerät erreichen.

Zur Vermeidung gefährlicher Drücke in der Lunge wird die Inspirationszeit überwacht. In Fällen exzessiver Inspirationszeiten übernimmt ebenfalls das Beatmungsgerät selbständig wieder die Kontrolle, d.h. das Steuergerät wird abgeschaltet. In den Fällen, in denen die EKG-Impulse zu schnell auftreten, ist eine effektive Beatmung nicht länger garantiert. Auch in diesem Fall kann das Steuergerät sich automatisch abschalten und das konventionelle Beatmungsgerät übernimmt selbständig die weitere Beatmung. Im Falle der zu kurz aufeinanderfolgenden EKG-Impulse müssen jedoch mehrere solcher kurzzeitigen Impulse aufeinanderfolgen, um eine Abschaltung zu bewirken, da sie erst dann einen Dauerzustand signalisieren.

Zur Überwachung des zentralen Atemwegsdruckes ist vorgesehen, den Tubus nahe dem distalen Ende auf der Außenseite mit einem Drucksensor, insbesondere einem elektronischen Drucksensor, auszurüsten, der mit einer elektronischen Einrichtung zur Signalverarbeitung der erfaßten Meßwerte ausgerüstet ist. Des weiteren kann der elektronische Drucksensor den erfaßten Meßwert des zentralen Atemwegsdruckes in der Luftröhre an das Steuergerät liefern. Damit kann das Steuergerät zusätzlich zu den gegebenen Sicherheitsmechanismen die Funktion des Gesamtsystems kontrollieren und gegebenenfalls korrigieren. Weitere Details der Ausrüstung eines Tubus außenseitig mit einem Drucksensor sind in der Patentanmeldung DE-A-43 10 799.0 beschrieben. Darüber hinaus ist es möglich, mittels des mit dem Tubus mit integriertem Drucksensor fortlaufend in der Luftröhre ermittelten zentralen Atemwegsdruckes und der hieraus erhaltenen elektrischen Signale, die Beatmungsanlage in Abhängigkeit von dem herrschenden zentralen Atemwegsdruck des Patienten zu benutzen. Insbesondere ist es möglich, die dem ermittelten zentralen Atemwegsdruck entsprechenden elektrischen Signale der Beatmungsanlage zur Betätigung eines Triggers bei definiertem Druckabfall in der Beatmungsanlage einzuspeisen. Bei durchgehend kontrollierter Beatmung eines Patienten können die dem gemessenen zentralen Atemwegsdruck entsprechenden ermittelten elektrischen Signale in die Beatmungsanlage zur Steuerung der Funktion Druckbegrenzung bzw. obere Druckgrenze eingespeist werden, so daß die Beatmungsanlage bei Erreichen eines vorgegebenen Druckes entweder die Atemgaszufuhr abschaltet oder auf Ausatmung umschaltet. Insbesondere eignen sich kapazitive Drucksensoren mit integrierter elektronischer Signalverarbeitung, die als einbaufertige Baueinheit hergestellt werden. Die Drucksensoren arbeiten dabei nach dem Differenzprinzip, es werden also Druckunterschiede ermittelt und in elektronische Signale umgesetzt. Die analogen Signale der Meßgrößen werden mit Hilfe eines analog digital Umsetzers in digitale Signale umgesetzt, verstärkt und in Mikroprozessoren ausgewertet. Diese Elektronik ist als Chip ausgebildet und mit dem Drucksensor als Meßfühler zu einer Baueinheit kleiner Größe verbunden, beispielsweise 9 x 1,2 x 0,8 mm, so daß sie gut außenseitig auf der Tubuswandung angebracht oder eingesetzt werden können.

Das Steuergerät kann des weiteren erfindungsgemäß so ausgebaut werden, daß es in der Lage ist, das Exspirationsventil so zu steuern, daß ein definierter positiver Endausatmungsdruck (positive end expiratory pressure) PEEP am Patienten einstellbar ist.

Um den PEEP einzustellen, wird das Exspirationsventil nur teilweise geöffnet, so daß ein entsprechender gewisser Restdruck PEEP in den Atemwegen verbleibt.

Über die Bedienungselemente kann der PEEP in der Auswerte-Elektronik des Steuergerätes, siehe Figur 24, eingestellt werden. Die Auswerte-Elektronik ist in der Lage, den dann notwendigen Öffnungsgrad des Exspirationsventils zu berechnen. Diese Ausbaustufe für die Einstellung des PEEP ist bei allen Beatmungsanlagen, die ein regelbares Exspirationsventil aufweisen, anwendbar.

Bei Einsatz von Drucksensoren in Verbindung mit dem Tubus ist es möglich, das Steuergerät so auszubauen, daß es den exspiratorischen Druck mit Hilfe der Drucksensoren am exspiratorischen Schenkel des Beatmungsaufbaues messen kann und dieses Meßsignal in die Auswertung zur Erhöhung der Sicherheit des Gesamtsystems herangezogen wird. Des weiteren ist es auch möglich, mit Hilfe der Drucksensoren den eingestellten PEEP zu kontrollieren.

Während bisher bei konventionellen Beatmungsgeräten das Exspirationsventil mit der Atemgasquelle und den zugehörigen Bedienungs- und Einstellelementen eine Einheit bildete und das Steuergerät gemäß der Erfindung zusätzlich angeschlossen wird, ist es auch möglich, das erfindungsgemäße Steuergerät unmittelbar mit einem eigenen Exspirationsventil auszurüsten. Damit ist das Exspirationsventil zusammen mit dem Steuergerät unabhängig von der Beatmungsanlage und kann ebenfalls selbständig die Beatmung vornehmen.

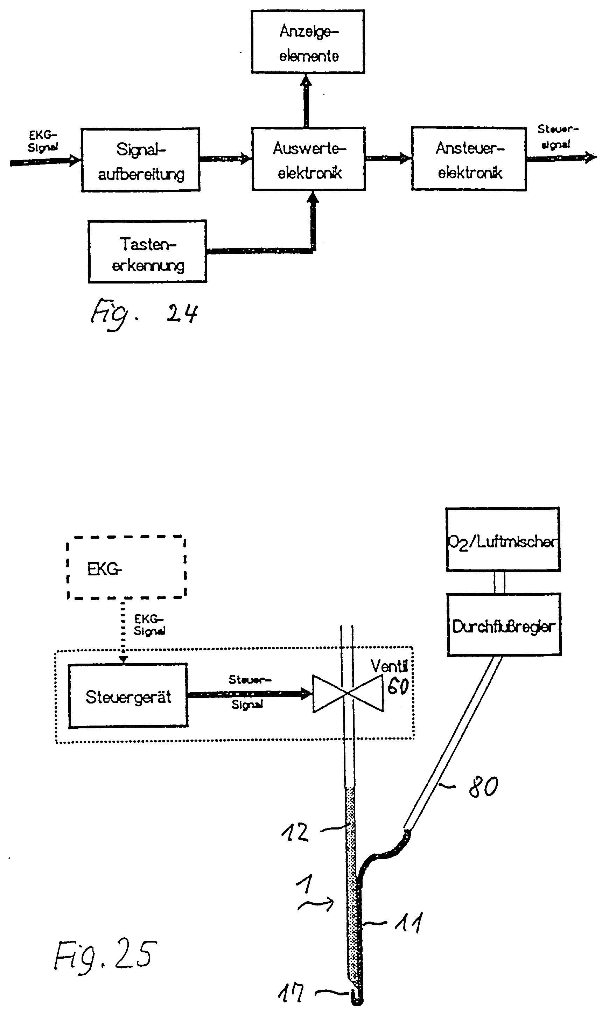

In der Figur 25 ist schematisch das Diagramm eines Steuergerätes mit internem Exspirationsventil 60 dargestellt. Der doppellumige Tubus 1 mit Respirationslumen 12 und Inspirationslumen sowie Düse 17 am Übergang des distalen Endes des Inspirationslumen zum Exspirationslumen wird mit Hilfe des Steuergerätes für eine pulssynchrone Beatmung mit einem retrograden Atemgasfluß eingesetzt. Mit einem Sauerstoff/Luftmischer wird aus der Sauerstoff/Luftversorgung das richtige Atemgasgemisch ausgewählt, mit dem Durchflußregler läßt sich der gewünschte Atemgasfluß einstellen und über die Leitung 80 dem Inspirationslumen 11 zugeführt. Das Steuergerät, das mittels der vom Elektrokardiographen EKG-Gerät erhaltenen EKG-Signale die Beatmung steuert, steuert das am proximalen Ausgang des Respirationslumens 12 angebrachte Exspirationsventil 60 unmittelbar. Eine Anordnung gemäß Figur 25 ermöglicht eine pulssynchrone Beatmung mit einem kontinuierlichen exspiratorischen retrograden Fluß nur mit einem Exspirationsventil, jedoch ohne ein konventionelles Beatmungsgerät.

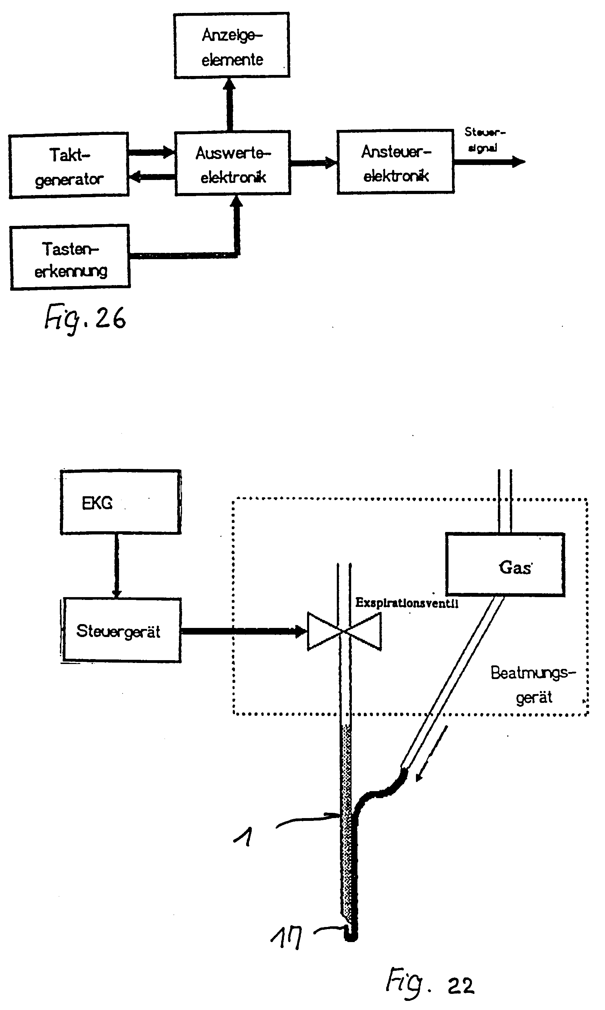

Um das Steuergerät vollständig unabhängig betreiben zu können, d.h. auch ohne EKG-Gerät oder Blutdruckmeßgerät wird erfindungsgemäß eine interne Takterzeugung, die dem Steuergerät zugeordnet ist, vorgesehen. Der Aufbau eines Steuergerätes mit interner Takterzeugung ist in Figur 26 im Blockdiagramm dargestellt. An Stelle eines Elektrokardiographen oder Blutdruckmeßgerätes ist ein Taktgenerator vorgesehen. Die durch den Taktgenerator erzeugten Impulse werden von der Auswerte-Elektronik erfaßt. Aus der Taktfrequenz und dem eingestellten I/E-Verhältnis berechnet die Auswerte-Elektronik den Zeitpunkt zum Öffnen und Schließen des Exspirationsventils. Über die Bedienungselemente und die Auswerte-Elektronik kann die Taktfrequenz zum Beispiel zwischen 2/Min. und 200/Min. eingestellt werden. Die Steuereinheit mit Taktgenerator kann beispielsweise in die Anlage gemäß Figur 25 integriert werden, so daß dort das noch vorhandene EKG-Gerät entfällt. Auf diese Weise ist es möglich, eine Anlage zur Beatmung zu schaffen, die als Stand-alone-Version, d.h. ohne konventionelles Beatmungsgerät einsetzbar ist. Bei Einsatz eines Taktgenerators in Verbindung mit dem Steuergerät, siehe Figur 26, ist auch eine nicht-pulssynchrone Beatmung mit einem kontinuierlichen exspiratorischen retrograden Atemgasfluß möglich.

Das erfindungsgemäße Steuergerät in den verschiedenen Ausbaustufen dient zur Steuerung der pulssychronen Beatmung mit einem kontinuierlichen retrograden Atemgasfluß in Verbindung mit einem doppellumigen Tubus, bei dem das Inspirationslumen am distalen Ende mit einer in die distale Austrittsöffnung des Respirationslumens hineinblasenden Düse ausgestattet ist. Mit Hilfe des Steuergerätes wird ein Exspirationsventil, das entweder extern in einem handelsüblichen Beatmungsgerät vorhanden ist oder intern direkt mit dem Steuergerät verbunden ist, siehe Figur 25, geöffnet und geschlossen. Die Ansteuerung des Exspirationsventiles erfolgt pulssynchron, so daß die Inspiration entweder in der Systole oder in der Diastole erfolgt. Um die Phasen des Herzens zu erfassen, wird das Steuergerät mit einem EKG-Gerät oder einem Blutdruckmeßgerät verbunden.

Das Steuergerät ist so konzipiert, daß es auch als Stand-alone-Gerät mit integriertem Exspirationsventil und eigener Takterzeugung (asynchron), siehe Figur 26, betrieben werden kann. Zusätzlich kann mit diesem Gerät der Restdruck in den Atemwegen eingestellt werden. Auch die Möglichkeit zur direkten Messung und Steuerung des zentralen Atemwegsdruckes mittels Drucksensoren ist möglich.

## Patentansprüche

1. Tubus (1) zum Einführen in die Atemwege eines Patienten insbesondere orotracheale oder nasotracheale Tuben oder Tracheostomie-Tuben für eine steuerbare Beatmung eines Patienten mit einem distalen in die Atemwege einführbaren Ende und einem proximalen mit Anschlüssen versehenen Ende und zwei im wesentlichen vom proximalen bis zum distalen Ende des Tubus durchgehenden Lumina (11, 12), bei dem das eine der beiden Lumen als Respirationslumen bei der Beatmung oder Atmung und das andere der beiden Lumen als Inspirationslumen der zusätzlichen oder alleinigen Zufuhr von Atemgas dient, **dadurch gekennzeichnet**, daß jedes der beiden Lumen eine distale Austrittsöffnung aufweist und die distale Austrittsöffnung des Inspirationslumens (11) auf die distale Austrittsöffnung (123) des Respirationslumens (12) gerichtet ist.

2. Tubus nach Anspruch 1,
**dadurch gekennzeichnet**, daß eines der beiden Lumen einen kleineren Querschnitt als das andere der beiden Lumen des Tubus aufweist und das Lumen mit dem gegenüber dem anderen Lumen größeren Querschnitt als Respirationslumen für die Beatmung und Atmung und das Lumen mit dem gegenüber dem anderen Lumen kleineren Querschnitt als Inspirationslumen für die zusätzliche oder alleine Atemgaszufuhr dient.

3. Tubus nach Anspruch 1 oder 2,
**dadurch gekennzeichnet**, daß das distale Ende des Inspirationslumens (11) zu einer Düse verengt ist und in einer auf die distale Austrittsöffnung (123) des Respirationslumens (12) gerichteten Düsenaustrittsöffnung (171) mündet.

4. Tubus nach einem der Ansprüche 1 bis 3,
**dadurch gekennzeichnet**, daß das Respirationslumen der Einführung eines Absaugkatheters oder Bronchoskopes dient.

5. Tubus nach Anspruch 1,
**dadurch gekennzeichnet**, daß eines der beiden Lumen einen kleineren Querschnitt als das andere der beiden Lumen des Tubus aufweist und das Lumen mit dem gegenüber dem anderen Lumen größeren Querschnitt als Respirationslumen für die Beatmung und Atmung und das Lumen mit dem gegenüber dem anderen Lumen kleineren Querschnitt als Inspirationslumen für die zusätzliche oder alleinige Atemgaszufuhr dient und das distale Ende des Inspirationslumens (11) zu einer Düse verengt ist und in einer auf die distale Austrittsöffnung (123) des Respirationslumens (12) gerichteten Düsenaustrittsöffnung (171) mündet, wobei die Größe der Querschnitte von Respirationslumen,Inspirationslumen und Düsenaustrittsöffnung sich wie etwa 8:2:1 verhalten.

6. Tubus nach einem der Ansprüche 1 bis 5,
**dadurch gekennzeichnet**, daß das distale Ende des Inspirationslumens (11) im Bereich des distalen Endes (19) des Tubus (1) um etwa 180° umgelenkt ist und auf die distale Austrittsöffnung (123) des Respirationslumens (12) und durch das Respirationslumen (12) zum proximalen Ende des Tubus (1) gerichtet ist.

7. Tubus nach Anspruch 1 oder 2,
**dadurch gekennzeichnet**, daß die distale Austrittsöffnung (123) des Respirationslumens (12) schräg zur Längsachse (X) des Tubus verläuft, dergestalt, daß das distale Ende (19) des Tubus einseitig im Bereich des Respirationslumens zum Tubusende hin abgeschrägt ist.

8. Tubus nach einem der Ansprüche 1, 5, 6 oder 7,
**dadurch gekennzeichnet**, daß die Düsenaustrittsöffnung (171) des Inspirationslumens (11) außermittig in bezug auf die Austrittssöffnung (123) des Respirationslumens nahe der Peripherie des Respirationslumens (12) angeordnet ist.

9. Tubus nach einem der Ansprüche 3, 5, 6, 7 oder 8,
**dadurch gekennzeichnet**, daß die Düsenaustrittsöffnung (171) des Inspirationslumens (11) und die distale Austrittsöffnung (123) des Respirationslumens (12) in der Weise aneinandergrenzen, daß dort, wo die Austrittsöffnung (123) des Respirationslumens endet, die Düsenaustrittsöffnung (171) des Inspirationslumens angeordnet ist.

10. Tubus nach einem der Ansprüche 3, 5, 6, 7 oder 8,
**dadurch gekennzeichnet**, daß die Düsenaustrittsöffnung (171) des Inspirationslumens außerhalb der distalen Austrittsöffnung (123) des Respirationslumens und außerhalb des Respirationslumens angeordnet ist.

11. Tubus nach einem der Ansprüche 3, 5, 6, 7 oder 8,
**dadurch gekennzeichnet**, daß die Düsenaustrittsöffnung (171) des Inspirationslumens teilweise in das Respirationslumen (12) durch die distale Austrittsöffnung (123) des Respirationslumens (12) hineinragt.

12. Tubus nach einem der Ansprüche 1 bis 11,
**dadurch gekennzeichnet**, daß das Respirationslumen (12) und das Inspirationslumen (11) durch eine Trennwand (14) in dem Tubus (1) voneinander abgeteilt sind.

13. Tubus nach einem der Ansprüche 3 und 5 bis 12,
**dadurch gekennzeichnet**, daß zur Ausbildung der düsenförmigen Verengung des Inspirationslumens und der Düsenaustrittsöffnung (171) am distalen Ende (19) des Tubus (1) ein Düsenformstück (9) mit dem Tubus in Verlängerung des Inspirationslumens (11) unter Ausbildung eines das Inspirationslumen (11) fortsetzenden Düsenkanals (17a) verbunden ist.

14. Tubus nach Anspruch 13,
**dadurch gekennzeichnet**, daß das Düsenformstück (9) zweifach rechtwinklig abgewinkelt ist, wodurch der Strömungsweg des Inspirationslumens (11) um 180° umgelenkt wird.

15. Tubus nach Anspruch 14,
**dadurch gekennzeichnet**, daß die von dem Düsenformstück (9) gebildete Düsenaustrittsöffnung (171) für das Inspirationslumen (11) in einer zur Abschrägung der Austrittsöffnung (123) des Respirationslumens (12) winkligen bis senkrechten Ebene abgeschrägt verläuft.

16. Tubus nach einem der Ansprüche 13 bis 15,
**dadurch gekennzeichnet**, daß das Düsenformstück (9) mit einem Schenkel (90) in das offene Ende am distalen Ende (19) des Tubus (1) des Inspirationslumens (11) eingesetzt und mit der Wandung des Tubus verrastet und/oder verklebt ist.

17. Tubus nach einem der Ansprüche 13 bis 15,
**dadurch gekennzeichnet**, daß das Düsenformstück (9) als Düsenring ausgebildet und am distalen Ende (19) des Tubus (1) aufgesteckt ist, wobei eine oder mehrere Düsenaustrittsöffnungen an der Innseite (96) des Düsenringes ausgebildet sind und das Respirationslumen (12) durch den Düsenring hindurchgeführt ist.

18. Tubus nach einem der Ansprüche 1 bis 17,
**dadurch gekennzeichnet**, daß ein Drucksensor (S) nahe dem distalen Ende (19) des Tubus (1) angebracht ist.

19. Tubus nach Anspruch 18,
**dadurch gekennzeichnet**, daß der nahe dem distalen Ende (19) des Tubus (1) angebrachte Drucksensor (S) außenseitig an bzw. in der Wandung des Tubus in einer Position angebracht ist, um Druck außerhalb des Tubus zu messen.

20. Tubus nach Anspruch 18 oder 19,
**dadurch gekennzeichnet**, daß ein elektronischer Drucksensor vorgesehen ist, der mit integrierter elektronischer Meßwerterfassung und Signalverarbeitung zum kontinuierlichen Messen des in der Luftröhre eines Patienten herrschenden zentralen Atemwegsdruckes ausgerüstet ist und der über eine Leitung mit einer Steuereinrichtung für ein Beatmungsgerät zum Steuern des Beatmungsgerätes in Abhängigkeit von dem in der Luftröhre eines Patienten herrschenden zentralen Atemwegsdruck verbindbar ist.

21. Tubus nach einem der Ansprüche 1 bis 20,
**dadurch gekennzeichnet**, daß nahe dem distalen Ende des Respirationslumens (12) der Querschnitt des Respirationslumens (12) auf einer kurzen Distanz in bezug auf die Längserstreckung des Exspirationslumens um 10 bis 25 % verkleinert ist.

22. Beatmungsanlage zur elektrisch steuerbaren maschinellen Beatmung eines Patienten mit einem Atemgas mit einem in die Atemwege eines Patienten einführbaren Tubus gemäß einem der vorangehenden Ansprüche, wobei das Respirationslumen des Tubus (1) an seinem proximalen Ende an ein steuerbares Exspirationsventil (60) und das Inspirationslumen (11) an seinem proximalen Ende an eine Atemgasquelle (5) für das Atemgas angeschlossen ist.

23. Beatmungsanlage nach Anspruch 22,
**dadurch gekennzeichnet**, daß ein elektrisches Steuergerat zum Steuern des Exspirationsventils (60) vorgesehen ist.

24. Beamtmungsanlage nach Anspruch 22 oder 23,
**dadurch gekennzeichnet**, daß das elektrische Steuergerät für das Exspirationsventil (60) an einen Elektrokardiographen oder ein Blutdruckmeßgerät angeschlossen ist und in Abhängigkeit von den gelieferten Ausgangssignalen des Elektrokardiographen bzw. Blutdruckmeßgerätes das Exspirationsventil (60) steuerbar ist, dergestalt, daß die Beatmung des Patienten mit dem über das Inspirationslumen (11) des Tubus (1) zugeführten Atemgas pulssynchron mit einem retrograden Fluß durchführbar ist.

25. Beatmungsanlage nach einem der Ansprüche 23 oder 24, **dadurch gekennzeichnet**, daß das Exspirationsventil (60) in Abhängigkeit von der Pulsfrequenz und von einem einstellbaren Inspirations-/Exspirationsverhältnis steuerbar ist.

26. Beatmungsanlage nach einem der Ansprüche 22 bis 25, **dadurch gekennzeichnet**, daß ein elektrisch steuerbares Druckminderventil (2) in der Zufuhrleitung für das Atemgas zum Inspirationslumen (11) des Tubus (1) vorgesehen ist, womit der Atemgasfluß in das Inspirationslumen während der Einatmung und Ausatmung frei wählbar und steuerbar ist.

27. Beatmungsanlage nach Anspruch 26,
**dadurch gekennzeichnet**, daß das elektrisch steuerbare Druckminderventil an das mit einem Elektrokardiographen oder Blutdruckmeßgerät verbundene elektronische Steuergerät angeschlossen ist und in Abhängigkeit der von dem Elektrokardiographen bzw. dem Blutdruckmeßgerät gelieferten Signale steuerbar ist.

28. Beatmungsanlage nach Anspruch 26 oder 27,
**dadurch gekennzeichnet**, daß der inspiratorische Atemgasfluß an das für die Kohlendioxidelimination erforderliche Atemminutenvolumen durch Steuerung des Druckminderventiles mit Hilfe des Steuergerätes anpaßbar ist und der exspiratorische Atemgasfluß an den für die Oxygenierung maßgeblichen mittleren Atemwegsdruck durch Steuerung des Druckminderventils mit Hilfe des Steuergerätes anpaßbar ist.

29. Beatmungsanlage nach einem der Ansprüche 23 bis 28, **dadurch gekennzeichnet**, daß das Steuergerät mit einer elektronischen Signalaufbereitung für das von dem Elektrokardiographen bzw. Blutdruckmeßgerät gelieferte elektrische Signal, einer elektronischen Auswertung für das aufbereitete empfangene Signal und einer elektronischen Ansteuerung für das an das Exspirationsventil (60) abzugebende Steuersignal sowie Bedienungselementen für Betriebsarten und Anzeigeelemente ausgestattet ist.

30. Beatmungsanlage nach einem der Ansprüche 23 bis 29, **dadurch gekennzeichnet**, daß das Steuergerät für das Exspirationsventil (60) Bedienungselemente für die Veränderung des zeitlichen Verhältnisses von Inspiration zu Exspiration sowie für die alternative Anwahl einer Synchronisierung der Einatmung mit der Kontraktion des Herzmuskels (systolische Inspiration) oder mit der Erschlaffung des Herzmuskels (diastolische Inspiration) aufweist.

31. Beatmungsanlage nach einem der Ansprüche 23 bis 30, **dadurch gekennzeichnet**, daß das Steuergerät automatisch bei Erkennung einer Fehlfunktion der maschinellen Beatmung oder des Elektrokardiographen abschaltbar ist.

32. Beatmungsanlage nach Anspruch 31,
**dadurch gekennzeichnet**, daß das Steuergerät an den in den Tubus integrierten Drucksensor gemäß einem der Ansprüche 18 bis 20 zur Überwachung des zentralen Atemwegsdruckes angeschlossen ist.

33. Beatmungsanlage nach einem der Ansprüche 23 bis 32, **dadurch gekennzeichnet**, daß ein definierter positiver Endausatmungsdruck (PEEP) am Patienten durch Steuerung des Exspirationsventils für eine teilweise Öffnung mittels des Steuergerätes einstellbar ist.

34. Beatmungsanlage nach einem der Ansprüche 23 bis 33, **dadurch gekennzeichnet**, daß der die Signale für das Steuergerät liefernde Elektrokardiograph bzw. Blutdruckmeßgerät durch einen entsprechende Signale liefernden Taktgenerator mit einstellbarer Taktfrequenz der Signale ersetzt ist.

35. Beatmungsanlage nach einem der Ansprüche 23 bis 34, **dadurch gekennzeichnet**, daß das Exspirationsventil Bestandteil eines konventionellen Beatmungsgerätes mit Atemgasquelle ist.

36. Beatmungsanlage nach einem der Ansprüche 23 bis 34, **dadurch gekennzeichnet**, daß das Steuergerät mit dem Exspirationsventil zu einer baulichen Einheit verbunden ist.

## Claims

1. Tube (1) for introduction into the respiratory tract of a patient, especially orotracheal or nasotracheal tubes or tracheostomy tubes for controllable ventilation of a patient, with a distal end that can be introduced into the respiratory tract and a proximal end provided with connections, and two passages (11, 12) extending essentially from the proximal to the distal ends of the tube, with one of the two passages serving as the ventilation passage during ventilation or breathing and the other of the two passages serving as the inspiration passage for additional or sole administration of fresh gas, characterized by the fact that each of the two passages has a distal outlet opening and the distal outlet opening of inspiration passage (11) is directed at the distal outlet opening (123) of ventilation passage (12).

2. Tube according to Claim 1 characterized by the fact that one of the two passages has a smaller cross section than the other of the two passages of the tube, and the passage with the cross section that is larger relative to the passage of the other serves as the ventilation passage for ventilation and breathing and the passage with the cross section that is smaller than the other passage serves as an inspiration passage for the additional or sole fresh gas supply.

3. Tube according to Claim 1 or 2 characterized by the fact that the distal end of inspiration passage (11) is tapered to form a nozzle and terminates in a nozzle outlet opening (171) directed at the distal outlet opening (123) of ventilation passage (12).

4. Tube according to Claim 1 to 3 characterized by the fact that the ventilation passage serves for introduction of a suction catheter or bronchoscope.

5. Tube according to Claim 1 characterized by the fact that one of the two passages has a smaller cross section than the other of the two passages of the tube, and the passage with the cross section that is larger than that of the other passage serves as a ventilation passage for ventilation and breathing and the passage with the cross section that is smaller than that of the other passage serves as the inspiration passage for the additional or sole fresh gas supply, and the distal end of inspiration passage (11) is tapered to form a nozzle and terminates in a nozzle outlet opening (171) directed at the distal outlet opening (123) of ventilation passage (12), with the size of the cross section of the ventilation passage, inspiration passage, and nozzle outlet opening having ratios of approximately 8:2:1 to one another.

6. Tube according to one of Claims 1 to 5 characterized by the fact that the distal end of inspiration passage (11) is bent in the vicinity of the distal end (19) of tube (1) by approximately 180° and is directed at the distal outlet opening (123) of ventilation passage (12) and through ventilation passage (12) to the proximal end of tube (1).

7. Tube according to Claim 1 or 2 characterized by the fact that the distal outlet opening (123) of ventilation passage (12) runs diagonally to lengthwise axis (X) of the tube, in such manner that the distal end (19) of the tube is beveled unilaterally in the vicinity of the ventilation passage toward the end of the tube.

8. Tube according to one of Claims 1, 5, 6, or 7 characterized by the fact that nozzle outlet opening (171) of inspiration passage (11) is located off center relative to outlet opening (123) of the ventilation passage near the periphery of ventilation passage (12).

9. Tube according to one of Claims 3, 5, 6, 7, or 8 characterized by the fact that nozzle outlet opening (171) of inspiration passage (11) and distal outlet opening (123) of ventilation passage (12) abut one another in such fashion that at the point where outlet opening (123) of ventilation passage terminates, nozzle outlet opening (171) of the inspiration passage is located.

10. Tube according to one of Claims 3, 5, 6, 7, or 8 characterized by the fact that nozzle outlet opening (171) of the inspiration passage is located outside distal outlet opening (123) of the ventilation passage and outside the ventilation passage.

11. Tube according to one of Claims 3, 5, 6, 7, or 8 characterized by the fact that nozzle outlet opening (171) of the inspiration passage partially projects into ventilation passage (12) through distal outlet opening (123) of ventilation passage (12).

12. Tube according to one of Claims 1 to 11 characterized by the fact that ventilation passage (12) and inspiration passage (11) are divided from one another by a partition (14) in tube (1).

13. Tube according to one of Claims 3 and 5 to 12 characterized by the fact that, to form the nozzle-shaped taper of the inspiration passage and nozzle outlet opening (171) at the distal end (19) of tube (1), a nozzle-shaped piece (9) is connected with the tube as an extension of inspiration passage (11), forming a nozzle channel (17a) which continues inspiration passage (11).

14. Tube according to Claim 13 characterized by the fact that nozzle-shaped piece (9) is bent at right angles twice, so that the flow path of inspiration passage (11) is deflected through 180°.

15. Tube according to Claim 14 characterized by the fact that nozzle outlet opening (171) formed by nozzle-shaped piece (9) for inspiration passage (11) runs beveled in a plane which is at an angle to or perpendicular to the bevel of outlet opening (123) of ventilation passage (12).

16. Tube according to one of Claims 13 to 15 characterized by the fact that nozzle-shaped piece (9) is inserted with one leg (90) into the open end of inspiration passage (11) at the distal end (19) of tube (1) and is latched and/or glued to the wall of the tube.

17. Tube according to one of Claims 13 to 15 characterized by the fact that nozzle-shaped piece (9) is made in the form of a nozzle ring and fitted on the distal end (19) of tube (1), with one or more nozzle outlet openings being formed on the inside (96) of the nozzle ring and the ventilation passage (12) running through the nozzle ring.

18. Tube according to one of Claims 1 to 17 characterized by the fact that a pressure sensor (S) is mounted near distal end (19) of tube (1).

19. Tube according to Claim 18 characterized by the fact that pressure sensor (S) mounted near distal end (19) of tube (1) is mounted externally on or in the wall of the tube in a position for measuring the pressure outside the tube.

20. Tube according to Claim 18 or 19 characterized by the fact that an electronic pressure sensor is provided, equipped with integrated electronic measured value detection and signal processing for continuous measurement of the central airway pressure prevailing in the trachea of a patient, which can be connected through a line with a control device for a ventilator to control the ventilator as a function of the central airway pressure prevailing in the trachea of a patient.

21. Tube according to one of Claims 1 to 20 characterized by the fact that near the distal end of ventilation passage (12), the cross section of ventilation passage (12) is reduced by 10 to 25% for a short distance relative to the lengthwise extent of exhalation passage.

22. Ventilator system for electrically controllable mechanical ventilation of a patient with fresh gas, with a tube insertable into the respiratory tract of a patient, according to one of the previous claims, with the ventilation passage of tube (1) being connected at its proximal end to a controllable expiration valve (60) and with inspiration passage (11) being connected at its proximal end to a fresh gas source (5).

23. Ventilator system according to Claim 22 characterized by the fact that an electrical control device is provided to control expiration valve (60).

24. Ventilator system according to Claim 22 or 23 characterized by the fact that the electrical control device for expiration valve (60) is connected to an electrocardiograph or blood pressure measuring device and, depending on the output signals delivered by the electrocardiograph or blood pressure measuring device, expiration valve (60) is controllable in such fashion that ventilation of the patient with fresh gas supplied through inspiration passage (11) of tube (1) can be performed, synchronized with the pulse, with a retrograde flow.

25. Artificial ventilator according to one of Claims 23 or 24 characterized by the fact that expiration valve (60) is controllable as a function of the pulse frequency and an adjustable inhalation/exhalation ratio.

26. Ventilator system according to one of Claims 22 to 25 characterized by the fact that an electrically controllable pressure-reduction valve (2) is provided in the supply line for the fresh gas to inspiration passage (11) of tube (1), with the fresh gas flow into the inspiration passage being freely selectable and controllable during inhalation and exhalation.

27. Ventilator system according to Claim 26 characterized by the fact that the electrically controllable pressure-reduction valve is connected to the electronic control device connected to the electrocardiograph or blood pressure measuring device, and is controllable as a function of the signals delivered by the electrocardiograph or blood pressure measuring device.

28. Ventilator system according to Claim 26 or 27 characterized by the fact that the inhalational fresh gas flow is adjustable to the minute volume required for carbon dioxide elimination by controlling the pressure-reduction valve with the aid of a control device, and the exhalational gas flow is adjustable to the mean airway pressure that is critical for oxygenation by controlling the pressure-reduction valve with the aid of the control device.

29. Ventilator system according to one of Claims 22 or 28 characterized by the fact that the control device is equipped with electronic signal preparation for the electrical signal delivered by the electrocardiograph or blood pressure measuring device, and with electronic evaluation for the processed received signal and electronic control for the control signal to be delivered to expiration valve (60), as well as operating elements for operating modes and display elements.

30. Ventilator system according to one of Claims 23 or 29 characterized by the fact that the control device for expiration valve (60) has operating elements for changing the time ratio from inhalation to exhalation as well for the alternative selection of synchronization of inhalation with contraction of the heart muscle (systolic inhalation) or with relaxation of the heart muscle (diastolic inhalation).

31. Ventilator system according to one of Claims 23 to 30 characterized by the fact that the control device is automatically switchable upon recognition of improper functioning of mechanical ventilation or of the electrocardiograph.

32. Ventilator system according to Claim 31 characterized by the fact that the control device is connected to the pressure sensor integrated into the tube according to one of Claims 18 to 20 to monitor the central airway pressure.

33. Ventilator system according to one of Claims 23 to 32 characterized by the fact that a defined positive end-expiratory pressure (PEEP) for the patient can be adjusted by controlling the expiration valve for partial opening by means of the control device.

34. Ventilator system according to one of Claims 23 to 33 characterized by the fact that the electrocardiograph or blood pressure measuring device supplying signals for the control device is replaced by a clock supplying corresponding signals with adjustable clock frequency of the signals.

35. Ventilator system according to one of Claims 23 to 34 characterized by the fact that the expiration valve is part of a conventional ventilator with a fresh gas source.

36. Ventilator system according to one of Claims 23 to 34 characterized by the fact that the control device is connected with the expiration valve to form a structural unit.

## Revendications

1. Tube trachéal (1) à introduire dans les voies respiratoires d'un patient, en particulier tubes orotrachéaux ou nasotrachéaux ou tubes de trachéotomie pour la mise d'un patient sous assistance respiratoire, comportant une extrémité distale qu'on peut introduire dans les voies respiratoires et une extrémité proximale pourvue de raccords, et deux tubulures (11, 12) s'étendant pratiquement de l'extrémité proximale à l'extrémité distale du tube trachéal, pour lequel une des deux tubulures est employée comme tubulure d'expiration lors de la ventilation artificielle ou de la respiration et l'autre des deux tubulures est employée comme tubulure d'inspiration pour l'apport, à titre complémentaire ou exclusif, de gaz respiratoire, caractérisé en ce que chacune des deux tubulures présente une ouverture de sortie distale et en ce que l'ouverture de sortie distale de la tubulure d'inspiration (11) est orientée en direction de l'ouverture de sortie distale (123) de la tubulure d'expiration (12).

2. Tube trachéal suivant la revendication 1, caractérisé en ce qu'une des deux tubulures du tube trachéal présente une section plus faible que l'autre des deux tubulures et en ce que la tubulure présentant une section plus grande que celle de l'autre tubulure est employée comme tubulure d'expiration pour la ventilation artificielle et la respiration et en ce que la tubulure présentant une section plus petite que celle de l'autre tubulure est employée comme tubulure d'inspiration pour l'apport complémentaire ou exclusif de gaz respiratoire.

3. Tube trachéal suivant la revendication 1 ou 2, caractérisé en ce que l'extrémité distale de la tubulure d'inspiration (11) se rétrécit pour former une buse et se termine par une ouverture de sortie de buse (171) orientée vers l'ouverture de sortie distale (123) de la tubulure d'expiration (12).

4. Tube trachéal suivant l'une des revendications 1 à 3, caractérisé en ce que la tubulure d'expiration sert à l'introduction d'un cathéter d'aspiration ou d'un bronchoscope.

5. Tube trachéal suivant la revendication 1, caractérisé en ce qu'une des deux tubulures du tube trachéal présente une section plus faible que l'autre des deux tubulures et en ce que la tubulure présentant une section plus grande que celle de l'autre tubulure est employée comme tubulure d'expiration pour la ventilation artificielle et la respiration et en ce que la tubulure présentant une section plus petite que celle de l'autre tubulure est employée comme tubulure d'inspiration pour l'apport complémentaire ou exclusif de gaz respiratoire et en ce que l'extrémité distale de la tubulure d'inspiration (11) se rétrécit pour former une buse et se termine par une ouverture de buse (171) orientée vers l'ouverture de sortie distale (123) de la tubulure d'expiration (12), la grandeur de la section de la tubulure d'expiration, de la tubulure d'inspiration et de l'ouverture de sortie de la buse sont dans un rapport d'environ 8:2:1.

6. Tube trachéal suivant l'une des revendications 1 à 5, caractérisé en ce que l'extrémité distale de la tubulure d'inspiration (11) est recourbée à environ 180° à proximité de l'extrémité distale (19) du tube trachéal (1) et est orientée vers l'ouverture de sortie distale (123) de la tubulure d'expiration (12) du tube trachéal (1).

7. Tube trachéal suivant la revendication 1 ou 2, caractérisé en ce que l'ouverture de sortie distale (123) de la tubulure d'expiration (12) s'étend en oblique par rapport à l'axe longitudinal (X) du tube trachéal, de telle manière que l'extrémité distale (19) du tube trachéal est biseautée d'un côté vers l'extrémité du tube trachéal à proximité de la tubulure d'expiration.

8. Tube trachéal suivant l'une des revendications 1, 5, 6 ou 7, caractérisé en ce que l'ouverture de sortie de la buse (171) de la tubulure d'inspiration (11) est disposée à l'extérieur et de manière centrale par rapport à l'ouverture de sortie (123) de la tubulure d'expiration, près de la périphérie de la tubulure d'expiration (12).

9. Tube trachéal suivant l'une des revendications 3, 5, 6, 7 ou 8, caractérisé en ce que l'ouverture de sortie de la buse (171) de la tubulure d'inspiration (11) et l'ouverture de sortie distale (123) de la tubulure d'expiration (12) se touchent de telle manière que l'ouverture de sortie de la buse (171) de la tubulure d'inspiration (11) est disposée là où se termine l'ouverture de sortie distale (123) de la tubulure d'expiration.

10. Tube trachéal suivant l'une des revendications 3, 5, 6, 7 ou 8, caractérisé en ce que l'ouverture de sortie de la buse (171) de la tubulure d'inspiration est disposée en-dehors de l'ouverture de sortie distale (123) de la tubulure d'expiration et endehors de la tubulure d'expiration.

11. Tube trachéal suivant l'une des revendications 3, 5, 6, 7 ou 8, caractérisé en ce que l'ouverture de sortie de la buse (171) de la tubulure d'inspiration pénètre en partie dans la tubulure d'expiration (12) à travers l'ouverture de sortie distale (123) de la tubulure d'expiration (12).

12. Tube trachéal suivant l'une des revendications 1 à 11, caractérisé en ce que la tubulure d'expiration (12) et la tubulure d'inspiration (11) sont séparées l'une de l'autre par une paroi de séparation (14) dans le tube trachéal (1).

13. Tube trachéal suivant l'une des revendications 3 et 5 à 12, caractérisé en ce que, pour former le rétrécissement en forme de buse de la tubulure d'inspiration et l'ouverture de sortie de la buse (171) à l'extrémité distale (19) du tube trachéal (1), une pièce en forme de buse (9) est liée au tube trachéal à titre de prolongation de la tubulure d'inspiration (11) en formant un canal de buse (17a) prolongeant la tubulure d'inspiration (11).

14. Tube trachéal suivant la revendication 13, caractérisé en ce que la pièce en forme de buse (9) présente deux coudes à angle droit, ce qui provoque une déviation à 180° du chemin d'écoulement de la tubulure d'inspiration (11).

15. Tube trachéal suivant la revendication 14, caractérisé en ce que l'ouverture de sortie de la buse (171) formée par la pièce en forme de buse (9) pour la tubulure d'inspiration (11) s'étend dans un plan oblique à vertical pour former l'inclinaison de l'ouverture de sortie (123) de la tubulure d'expiration (12).

16. Tube trachéal suivant l'une des revendications 13 à 15, caractérisé en ce que la pièce en forme de buse (9) est introduite par une branche (90) dans l'extrémité ouverte de la tubulure d'inspiration (11) à l'extrémité distale (19) du tube trachéal (1) et est verrouillée/collée à la paroi du tube trachéal.

17. Tube trachéal suivant l'une des revendications 13 à 15, caractérisé en ce que la pièce en forme de buse (9) a la forme d'un anneau de buse et est placée sur l'extrémité distale (19) du tube trachéal (1), une ou plusieurs ouvertures de sortie de buse étant formées à la face interne (96) de l'anneau de buse et la tubulure d'expiration (12) étant insérée à travers l'anneau de buse.

18. Tube trachéal suivant l'une des revendications 1 à 17, caractérisé en ce qu'un capteur de pression (S) est disposé près de l'extrémité distale (19) du tube trachéal (1).

19. Tube trachéal suivant la revendication 18, caractérisé en ce que le capteur de pression (S) disposé près de l'extrémité distale (19) du tube trachéal (1) est placé extérieurement à la paroi ou dans la paroi du tube trachéal dans une position appropriée pour mesurer la pression à l'extérieur du tube trachéal.

20. Tube trachéal suivant la revendication 18 ou 19, caractérisé en ce qu'on prévoit un capteur de pression électronique, équipé d'un système électronique intégré de détermination de la valeur de mesure et de traitement du signal, pour la mesure en continu de la pression des voies respiratoires centrales régnant dans la trachée d'un patient et qui peut être relié via une tuyauterie à un dispositif de commande pour appareil respiratoire afin de commander l'appareil respiratoire en fonction de la pression des voies respiratoires centrales régnant dans la trachée d'un patient.

21. Tube trachéal suivant l'une des revendications 1 à 20, caractérisé en ce que, près de l'extrémité distale de la tubulure d'expiration (12), la section de la tubulure d'expiration (12) est réduite de 10 à 25% sur une courte distance par rapport à la longueur totale de la tubulure d'expiration.

22. Installation de ventilation artificielle pour la ventilation artificielle d'un patient à l'aide d'une machine à commande électrique, avec un gaz respiratoire, via un tube trachéal pouvant être introduit dans les voies respiratoires d'un patient, conformément à une des revendications précédentes, la tubulure d'expiration du tube trachéal (1) étant raccordée par son extrémité proximale à une vanne d'expiration (60) pouvant être commandée et la tubulure d'inspiration (11) étant raccordée par son extrémité proximale à une source de gaz respiratoire (5) pour le gaz respiratoire.

23. Installation de ventilation artificielle suivant la revendication 22, caractérisée en ce qu'un appareil électrique de commande est prévu pour commander la vanne d'expiration (60).

24. Installation de ventilation artificielle suivant l'une des revendications 22 ou 23, caractérisée en ce que l'appareil électrique de commande pour la vanne d'expiration (60) est raccordé à un électrocardiographe ou un appareil de mesure de la tension artérielle et en ce que la vanne d'expiration (60) peut être commandée en fonction des signaux de sortie de l'électrocardiographe ou de l'appareil de mesure de la tension artérielle, de telle manière à pouvoir réaliser la ventilation artificielle du patient avec le gaz respiratoire amené par la tubulure d'inspiration (11) du tube trachéal (1) en synchronisme d'impulsion avec un flux rétrograde.

25. Installation de ventilation artificielle suivant une des revendications 23 ou 24, caractérisée en ce qu'on peut commander la vanne d'expiration (60) en fonction de la fréquence du pouls et d'un rapport réglable inspiration/expiration.

26. Installation de ventilation artificielle suivant l'une des revendications 22 à 25, caractérisée en ce qu'une vanne de détente à commande électrique (2) est prévue dans la tuyauterie d'amenée du gaz respiratoire dans la tubulure d'inspiration (11) du tube trachéal (1), grâce à laquelle on peut sélectionner et commander librement le débit de gaz respiratoire dans la tubulure d'inspiration pendant l'inspiration et l'expiration.

27. Installation de ventilation artificielle suivant la revendication 26, caractérisée en ce que la vanne de détente à commande électrique est raccordée à l'appareil électronique de commande raccordé à un électrocardiographe ou un appareil de mesure de la tension artérielle et peut être commandée en fonction des signaux fournis par l'électrocardiographe ou l'appareil de mesure de la tension artérielle.

28. Installation de ventilation artificielle suivant la revendication 26 ou 27, caractérisée en ce qu'on peut adapter le débit de gaz respiratoire inspiré au volume respiratoire par minute nécessaire pour l'élimination du dioxyde de carbone par commande de la vanne de détente à l'aide de l'appareil de commande et en ce qu'on peut adapter le débit de gaz de respiration expiré à la pression moyenne des voies respiratoires déterminante pour l'oxygénation par commande de la vanne de détente à l'aide de l'appareil de commande.

29. Installation de ventilation artificielle suivant l'une des revendications 23 à 28, caractérisée en ce que l'appareil de commande est équipé d'une installation électronique de traitement des signaux pour le signal électrique fourni par l'électrocardiographe ou l'appareil de mesure de la tension artérielle, d'un système électronique d'évaluation du signal reçu après traitement et d'une commande électronique pour le signal de commande à envoyer vers la vanne d'expiration (60) ainsi que d'éléments de manoeuvre pour les modes de fonctionnement et les éléments d'affichage.

30. Installation de ventilation artificielle suivant l'une des revendications 23 à 29, caractérisée en ce que l'appareil de commande pour la vanne d'expiration (60) présente des éléments de manoeuvre pour la modification des rapports temporels entre inspiration et expiration ainsi que pour la sélection alternative d'une synchronisation de l'inspiration avec la contraction du muscle cardiaque (inspiration systolique) ou avec la détente du muscle cardiaque (inspiration diastolique).

31. Installation de ventilation artificielle suivant l'une des revendications 23 à 30, caractérisée en ce que l'appareil de commande peut être mis automatiquement hors service en cas de détection d'un dysfonctionnement de la ventilation artificielle avec la machine ou de l'électrocardiographe.

32. Installation de ventilation artificielle suivant la revendication 31, caractérisée en ce que l'appareil de commande est raccordé au capteur de pression qui est intégré au tube trachéal conformément à une des revendications 18 à 20 pour la surveillance de la pression des voies respiratoires centrales.

33. Installation de ventilation artificielle suivant l'une des revendications 23 à 32, caractérisée en ce qu'on peut régler au moyen de l'appareil de commande une pression positive définie de fin d'expiration (PEEP) du patient par commande d'ouverture partielle de la vanne d'expiration.

34. Installation de ventilation artificielle suivant l'une des revendications 23 à 33, caractérisée en ce que l'électrocardiographe ou l'appareil de mesure de la tension artérielle fournissant les signaux pour l'appareil de commande est remplacé par un générateur d'impulsions à fréquence réglable d'impulsion des signaux fournissant des signaux correspondants.

35. Installation de ventilation artificielle suivant l'une des revendications 23 à 34, caractérisée en ce que la vanne d'expiration fait partie d'un appareil de ventilation artificielle conventionnel avec source de gaz respiratoire.

36. Installation de ventilation artificielle suivant l'une des revendications 23 à 34, caractérisée en ce que l'appareil de commande est combiné avec la vanne d'expiration de façon à former une unité intégrée.
